(19) Europäisches Patentamt / European Patent Office / Office européen des brevets

(11) EP 3 186 334 B1

(12) **EUROPÄISCHE PATENTSCHRIFT**

(45) Veröffentlichungstag und Bekanntmachung des Hinweises auf die Patenterteilung:
**25.09.2019 Patentblatt 2019/39**

(21) Anmeldenummer: **15750636.1**

(22) Anmeldetag: **10.08.2015**

(51) Int Cl.:
***C09K 19/60*** *(2006.01)* ***C09K 19/34*** *(2006.01)*
***C09B 5/62*** *(2006.01)* ***C09B 31/20*** *(2006.01)*
***C09B 57/00*** *(2006.01)*

(86) Internationale Anmeldenummer:
**PCT/EP2015/001643**

(87) Internationale Veröffentlichungsnummer:
**WO 2016/029996 (03.03.2016 Gazette 2016/09)**

(54) **VORRICHTUNG ZUR REGULIERUNG DES ENERGIE-DURCHTRITTS**

DEVICE FOR CONTROLLING THE PASSAGE OF ENERGY

DISPOSITIF POUR LA RÉGULATION DU PASSAGE DE L'ÉNERGIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.08.2014 EP 14002950**

(43) Veröffentlichungstag der Anmeldung:
**05.07.2017 Patentblatt 2017/27**

(73) Patentinhaber: **Merck Patent GmbH**
**64293 Darmstadt (DE)**

(72) Erfinder:
- **KIRSCH, Peer**
  **64342 Seeheim-Jugenheim (DE)**
- **BECK, Susann**
  **64283 Darmstadt (DE)**
- **JUNGE, Michael**
  **64319 Pfungstadt (DE)**
- **BEYER, Andreas**
  **63452 Hanau (DE)**
- **PATWAL, Ursula**
  **64354 Reinheim (DE)**

(56) Entgegenhaltungen:
**WO-A1-2013/102038 WO-A1-2014/090373**
**WO-A1-2015/090506**

- **JI QI ET AL: "Panchromatic small molecules for UV-Vis-NIR photodetectors with high detectivity", JOURNAL OF MATERIALS CHEMISTRY C: MATERIALS FOR OPTICAL AND ELECTRONIC DEVICES, Bd. 2, Nr. 13, 1. Januar 2014 (2014-01-01) , Seite 2431, XP055222959, UK ISSN: 2050-7526, DOI: 10.1039/c3tc32271h**
- **KMINEK IVAN ET AL: "Low-band gap copolymers containing thienothiadiazole units: synthesis, optical, and electrochemical properties", JOURNAL OF POLYMER SCIENCE PART A: POLYMER CHEMISTRY, JOHN WILEY & SONS, INC, US , Bd. 48, Nr. 13 1. Januar 2010 (2010-01-01), Seiten 2743-2756, XP008162027, ISSN: 0887-624X, DOI: DOI: 10.1002/POLA.24022 Gefunden im Internet: URL:http://onlinelibrary.wiley.com/doi/10.1002/pola.24022/pdf [gefunden am 2010-05-24]**
- **J. A. MIKROYANNIDIS ET AL: "Low band gap conjugated small molecules containing benzobisthiadiazole and thienothiadiazole central units: synthesis and application for bulk heterojunction solar cells", JOURNAL OF MATERIALS CHEMISTRY, Bd. 21, Nr. 12, 1. Januar 2011 (2011-01-01), Seite 4679, XP055062466, ISSN: 0959-9428, DOI: 10.1039/c0jm03436c**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 186 334 B1

## Beschreibung

Die vorliegende Erfindung betrifft eine Vorrichtung zur Regulierung des Energie-Durchtritts von einem Außenraum in einen Innenraum, Verbindungen, Fenster und Verwendungen der Vorrichtungen und Verbindungen.

Unter einer Vorrichtung zur Regulierung des Energie-Durchtritts wird vorliegend allgemein eine Vorrichtung verstanden, welche den Durchtritt von Energie durch eine Fläche reguliert, welche relativ gesehen hoch energiedurchlässig ist. Bevorzugt ist diese relativ gesehen hoch energiedurchlässige Fläche innerhalb einer relativ gesehen weniger energiedurchlässigen Struktur angeordnet. Beispielsweise kann die hoch energiedurchlässige Fläche eine Glasfläche oder eine offene Fläche sein, und die weniger energiedurchlässige Struktur, welche die hoch energiedurchlässige Fläche enthält, kann eine Wand sein.

Bevorzugt wird durch die Vorrichtung der Durchtritt von Energie aus Sonneneinstrahlung, entweder direkt oder indirekt, reguliert.

Der regulierte Energiedurchtritt erfolgt von einem Außenraum, bevorzugt der unmittelbar der Sonneneinstrahlung ausgesetzten Umwelt, in einen Innenraum, beispielsweise ein Gebäude oder ein Fahrzeug, oder eine andere von der Umgebung weitgehend abgeschlossene Einheit.

Unter dem Begriff Energie wird im Rahmen der vorliegenden Erfindung insbesondere Energie durch elektromagnetische Strahlung im UV-A-, VIS-, sowie NIR-Bereich verstanden. Insbesondere wird darunter Energie durch Strahlung verstanden, welche von den üblicherweise in Fenstern verwendeten Materialien (z. B. Glas) nicht oder nur in einem vernachlässigbaren Umfang absorbiert wird. Gemäß den üblicherweise verwendeten Definitionen wird unter dem UV-A-Bereich eine Wellenlänge von 320 bis 380 nm verstanden, unter dem VIS-Bereich eine Wellenlänge von 380 nm bis 780 nm verstanden und unter dem NIR-Bereich eine Wellenlänge von 780 nm bis 2000 nm verstanden. Entsprechend wird unter dem Begriff Licht allgemein elektromagnetische Strahlung mit Wellenlängen zwischen 320 und 2000 nm verstanden.

Unter einem dichroitischen Farbstoff wird im Rahmen der vorliegenden Erfindung eine lichtabsorbierende Verbindung verstanden, bei der die Absorptionseigenschaften von der Ausrichtung der Verbindung zur Polarisationsrichtung des Lichts abhängig sind. Typischerweise weist eine dichroitische Farbstoffverbindung eine langgestreckte Form auf, d.h. die Verbindung ist in einer Raumrichtung deutlich länger (Längsachse) als in den anderen beiden Raumrichtungen.

Auf dem Gebiet der Vorrichtungen zur Regulierung des Energie-Durchtritts von einem Außenraum in einen Innenraum sind in den vergangenen Jahren mehrere unterschiedliche technische Lösungen vorgeschlagen worden.

Eine vorteilhafte Lösung ist die Verwendung von Schaltschichten enthaltend ein flüssigkristallines Medium in Kombination mit einem oder mehreren dichroitischen Farbstoffen. Durch Anlegen einer Spannung kann bei diesen Schaltschichten eine Veränderung der räumlichen Ausrichtung der Moleküle der dichroitischen Verbindung erzielt werden, welche eine Änderung ihrer Absorption und damit der Transmission durch die Schaltschicht bewirkt. Eine entsprechende Vorrichtung ist beispielsweise in WO 2009/141295 beschrieben.

Alternativ kann eine solche Transmissionsänderung auch ohne elektrische Spannung durch einen Temperatur-induzierten Übergang von einem isotropen Zustand des flüssigkristallinen Mediums zu einem flüssigkristallinen Zustand erzielt werden, wie beispielsweise in US 2010/0259698 beschrieben.

WO 2015/090506 bildet Stand der Technik gemäß Artikel 54(3) EPÜ und beschreibt eine Vorrichtung zur Regulierung des Lichtdurchtritts, wobei die Vorrichtung eine Schicht umfasst, die ein flüssigkristallines Material, enthaltend eine Farbstoffverbindung und eine Verbindung mit einem bestimmten Strukturelement, enthält.

Weiterhin ist es bekannt, Vorrichtungen mit einer Schaltschicht, enthaltend ein flüssigkristallines Medium mit mindestens einem dichroitischen Farbstoff, so zu gestalten, dass die von dem Farbstoff absorbierte Energie teilweise als Fluoreszenzstrahlung wieder emittiert wird, die ihrerseits auf eine Solarzelle geleitet wird, welche sie in elektrische Energie umwandelt (WO 2009/141295).

Zur Verwendung in den genannten Vorrichtungen sind Rylen-Farbstoffe beschrieben worden, beispielsweise in der WO 2009/141295 und in WO 2014/090373.

Es ist besonders von Interesse, zu den genannten Zwecken fluoreszierende Farbstoffe einzusetzen, die für ihre hohe Lichtechtheit bekannt sind. Dazu zählen Perylen- oder Terrylenderivate, deren Stabilität allerdings den extremen Anforderungen für die Anwendung in Fenstern nicht genügt. Benzothiadiazol- und Diketopyrrolopyrrolderivate erfüllen zwar die Anforderungen im Hinblick auf Lichtechtheit und Dichroismus, absorbieren allerdings in der Regel zu kurzwellig, so dass sich der blaugrüne Farbbereich nur unzureichend abdecken lässt (Zhang et al., J. Material. Chem. 2004, 14, 1901 - 1904; WO2004/090046).

Die WO2010/031479A1, beschreibt ein Verfahren, bei dem Polymere mit Bis(thienocyclopenta)benzothiadiazol-Untereinheiten hergestellt werden. Die Polymere können als organische Halbleiter eingesetzt werden.

Die Synthese von Thienothiadiazolen wurde von Tanaka et al., 1994 (Heterocycles, 37, 2, 693-695) beschrieben. Thienothiadiazole können als Zwischenprodukte zur Synthese von photoaktiven Polymeren eingesetzt werden.

WO2013/102038, J. Qi et al. in Journal of Materials Chemistry C, 2014, 2, S. 2431-2438, J. Kminek et al. in Journal of Polymer Science A: Polymer Chemistry, 2010, 48, S. 2743-2756 und J. A. Mikroyannidis et al. in Journal of

Materials Chemistry, 2011, 21, S. 4679-4688 beschreiben Halbleiter mit Thienothiadiazol-Untereinheiten und deren Verwendungen, insbesondere als Transistoren und in Solarzellen. Die Verbindungen in WO 2013/102038 weisen im allgemeinen eine zentrale aromatische Struktureinheit auf, die an zwei Positionen mit Thienothiadiazol-Untereinheiten substituiert ist, die selbst mit weiteren aromatischen Struktureinheiten substituiert sind. Insgesamt weisen die Verbindungen im allgemeinen eine stabförmige Struktur auf, die zwei Thienothiadiazol-Untereinheiten aufweist. Zusätzlich werden zwei Verbindungen beschrieben, die jeweils nur eine Thienothiadiazol-Untereinheit aufweisen, und deren Verwendung als Elektronentransportmaterialien vorgeschlagen (Beispiele 15 bis 17). Anwendungen zur Regulierung des Energie-Durchtritts von einem Außenraum in einen Innenraum werden nicht offenbart.

Steinberger et al., 2012 (J. Mater. Chem., 22, 2701-2712) beschreiben die Herstellung von Verbindungen mit einer Thienothiadiazol-Untereinheit, und schlagen Verwendungen in Solarzellen in Kombination mit C60 Fullerenen vor. Anwendungen zur Regulierung des Energie-Durchtritts von einem Außenraum in einen Innenraum werden nicht offenbart.

Sharma et al., 2012 (RSC Advances, 2(30), 11457-11464) beschreiben eine Verbindung mit einer Thienothiadiazol-Untereinheit und Cyanoacrylsäure Substituenten, sowie deren Verwendung als Farbstoff.

Anwendungen zur Regulierung des Energie-Durchtritts von einem Außenraum in einen Innenraum werden nicht offenbart.

Bei den bekannten Vorrichtungen zur Regulierung des Energie-Durchtritts besteht hohes Interesse an der Entwicklung verbesserter Vorrichtungen und dazu geeigneter Verbindungen.

Der Erfindung liegt daher die Aufgabe zugrunde, neue, verbesserte Vorrichtungen und Verbindungen zur Regulierung des Energie-Durchtritts von einem Außenraum in einen Innenraum bereitzustellen, welche die oben beschriebenen Nachteile überwinden. Die Vorrichtungen und Verbindungen sollen dabei insbesondere in einer Schaltschicht einsetzbar sein.

Der Erfindung liegt dabei insbesondere die Aufgabe zugrunde, neue Farbstoffe mit starker Fluoreszenz, hoher Lichtechtheit, einem hohen dichroitischen Verhältnis und guter Löslichkeit in typischen Flüssigkristallmischungen bereitzustellen. Die Verbindungen sollen dabei die besonderen Anforderungen erfüllen, die sich bei Anwendungen im Zusammenhang mit Fenstern stellen, insbesondere in aktiven, flüssigkristall-basieren Abschattungsvorrichtungen.

Die Verbindungen sollen eine starke Lichtabsorption im VIS- und/oder NIR-Bereich des Lichts aufweisen. Der Erfindung liegt insbesondere die Aufgabe zugrunde, Vorrichtungen und Verbindungen bereitzustellen, die eine gute Absorption oberhalb 580 nm zeigen. Für Vorrichtungen, die emittiertes Fluoreszenzlicht in elektrische Energie umwandeln, ist es weiterhin von großem Interesse, dass die Verbindungen eine hohe Fluoreszenzquantenausbeute, eine hohe relative Fluoreszenz aus Wellenleitung und einen hohen Stokes-Shift aufweisen.

Die Vorrichtungen und Verbindungen sollen auch eine hohe Lebensdauer und einen hohen Schalthub aufweisen (d. h. Unterschieds der Transmission im Hellzustand zum Dunkelzustand). Weiterhin besteht bei Vorrichtungen, welche die Fluoreszenzemission der Farbstoffe zur Wiedergewinnung von Energie mittels einer Solarzelle nutzen, Verbesserungspotential bezüglich der Energieausbeute. Im optimalen Fall sollte die durch die Solarzelle bereitgestellte Energie ausreichen, um die gesamte für den Betrieb der Vorrichtung benötigte Energie bereitzustellen, bzw. sogar über diese Menge hinausgehen.

Überraschend wurde nun gefunden, dass die genannten technischen Aufgaben durch Vorrichtungen, Verbindungen, Fenster und Verwendungen gemäß den Patentansprüchen gelöst werden. Gegenstand der Erfindung ist eine Vorrichtung zur Regulierung des Energie-Durchtritts von einem Außenraum in einen Innenraum, wobei die Vorrichtung eine Schaltschicht enthält, wobei die Schaltschicht eine oder mehrere Verbindungen der Formel (I) enthält:

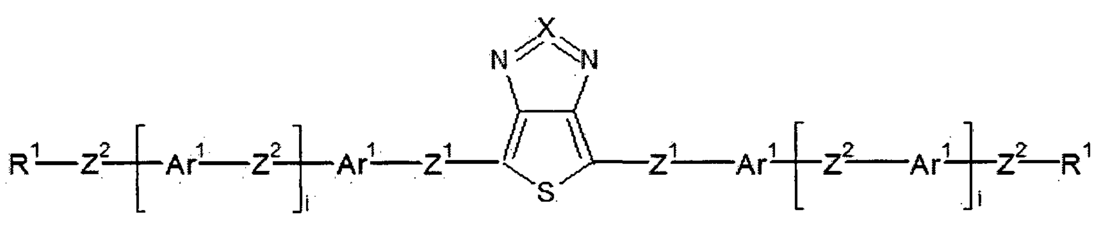

**Formel (I),**

wobei gilt:

X ist gleich S oder Se;

$Z^1$ ist unabhängig voneinander eine Einfachbindung, $-CR^3=CR^3-$ oder -C=C-; oder zwei, drei, vier oder fünf miteinander kombinierte Gruppen, gewählt aus den Gruppen $-CR^3=CR^3-$ und -C=C-;

$Z^2$ ist unabhängig voneinander eine Einfachbindung, O, S, $C(R^3)_2$, $-CR^3=CR^3-$ oder -C=C-; oder zwei, drei, vier oder fünf miteinander kombinierte Gruppen, gewählt aus den Gruppen O, S, $C(R^3)_2$, $-CR^3=CR^3-$ und -C≡C-;

$Ar^1$ ist unabhängig voneinander eine Aryl- oder Heteroarylgruppe mit 5 bis 30 aromatischen Ringatomen, die mit einem oder mehreren Resten $R^4$ substituiert sein kann;

$R^1$ ist unabhängig voneinander H, D, F, CN, $N(R^5)_2$, oder eine Alkyl-, Alkoxy- oder Thioalkoxygruppe mit 1 bis 20 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C=CR^5-$, -C≡C-, C=O, C=S, -C(=O)O-, -OC(=O)-, $Si(R^5)_2$, $NR^5$, -O- oder -S- ersetzt sein können;

$R^3$, $R^4$ sind unabhängig voneinander H, D, F, Cl, CN, oder eine Alkyl-, Alkoxy-, oder Thioalkoxygruppe mit 1 bis 10 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C=CR^5-$, -C≡C-, C=O, C=S, -C(=O)O-, -OC(=O)-, $Si(R^5)_2$, $NR^5$, -O- oder -S- ersetzt sein können;

$R^5$ ist unabhängig voneinander H, D, F, Cl, CN, $N(R^6)_2$, eine Alkyl-, Alkoxy- oder Thioalkoxygruppe mit 1 bis 10 C-Atomen oder eine Alkenyl- oder Alkinylgruppe mit 2 bis 20 C-Atomen, wobei die oben genannten Gruppen jeweils mit einem oder mehreren Resten $R^6$ substituiert sein können und wobei eine oder mehrere $CH_2$-Gruppen in den oben genannten Gruppen durch $-R^6C=CR^6-$, -C≡C-, C=O, C=S, -C(=O)O-, -O(C=O)-, $Si(R^6)_2$, $NR^6$, -O- oder -S- ersetzt sein können, oder eine Aryl- oder Heteroarylgruppe mit 5 bis 30 aromatischen Ringatomen, die jeweils mit einem oder mehreren Resten $R^6$ substituiert sein kann;

$R^6$ ist unabhängig voneinander H, F oder ein aliphatischer organischer Rest mit 1 bis 20 C-Atomen, in dem ein oder mehrere H-Atome durch F ersetzt sein können, oder eine Aryl- oder Heteroarylgruppe mit 5 bis 20 C-Atomen, in der ein oder mehrere H-Atome durch F ersetzt werden können;

i ist unabhängig voneinander gleich 0, 1, 2, 3, 4 oder 5,

und wobei neben der Verbindung der Formel (I) in der Schaltschicht ein flüssigkristallines Medium enthaltend eine oder mehrere verschiedene Verbindungen vorliegt.

Wenn i größer als 1 ist, können die Gruppen innerhalb der Klammern gleich oder verschieden sein.

Wenn i gleich 0 ist, fällt die Gruppe innerhalb der Klammern weg, und die Gruppen $Ar^1$ und $Z^2$ sind direkt miteinander verbunden.

Die Verbindung der Formel (I) ist ein Farbstoff. Dies bedeutet, dass sie im sichtbaren Bereich Licht zumindest teilweise absorbiert. Die Verbindung der Formel (I) ist bevorzugt dichroitisch. Dies bedeutet, dass sie Licht in Abhängigkeit von der Polarisation unterschiedlich stark absorbiert. Die Verbindung ist insbesondere ein dichroitischer Farbstoff.

Unter der Formulierung "zwei, drei, vier oder fünf miteinander kombinierte Gruppen, gewählt aus den Gruppen..." ist im Sinne der vorliegenden Erfindung zu verstehen, dass die Gruppen aneinander gebunden vorliegen, bevorzugt in Form einer Kette, in der zwei, drei, vier oder fünf der Gruppen aneinander gebunden vorliegen. Bevorzugt ist eine Kombination von genau zwei oder drei Gruppen. Die Gruppen können allgemein gleich oder verschieden sein.

Bevorzugt weist die Verbindung der Formel (I) an jeder Seite der zentralen Einheit zwei oder drei aromatische Gruppen auf. Die in Formel (I) dargestellten Gruppen, die substituiert sein können, bilden eine Kette.

In der Formel (I) können die gezeigten Gruppen allgemein gleich oder verschieden voneinander sein. Wenn also eine Verbindung der Formel (I) zwei oder mehr Gruppen aufweist, die in der Formel mit identischen Platzhaltern bezeichnet sind, wie beispielsweise $Ar^1$, so können die zwei oder mehr Gruppen $Ar^1$ gleich oder verschieden voneinander sein.

Eine Arylgruppe im Sinne dieser Erfindung enthält 6 bis 30 aromatische Ringatome; eine Heteroarylgruppe im Sinne dieser Erfindung enthält 5 bis 30 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (anellierter) aromatischer bzw. heteroaromatischer Polycyclus, beispielsweise Naphthalin, Phenanthren, Chinolin oder Carbazol verstanden. Ein kondensierter (anellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen. Ein solcher Polycyclus kann auch einzelne nichtkonjugierte Einheiten enthalten, wie es beispielsweise beim Fluoren-Grundkörper der Fall ist.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Fluoren, Spirobifluoren, Furan, Benzofuran, Isobenzofuran, Dibenzofuran, Thiophen, Benzothiophen, Isobenzothiophen, Dibenzothiophen, Benzodithiophen, Cyclopentadithiophen, Thienothiophen, Indenothiophen, Dithienopyrrol, Silolodithiophen, Selenophen, Benzoselenophen, Dibenzoselenophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Im Rahmen der vorliegenden Erfindung werden unter einer Alkylgruppe mit 1 bis 10 C-Atomen bzw. einer Alkenyl- oder Alkinylgruppe mit 2 bis 10 C-Atomen, in der auch einzelne H-Atome oder $CH_2$-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkoxygruppe mit 1 bis 10 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter einem aliphatischen organischen Rest mit 1 bis 20 C-Atomen wird prinzipiell ein beliebiger organischer Rest verstanden, welcher nicht aromatisch bzw. heteroaromatisch ist. Bevorzugt werden darunter Alkylgruppen mit 1 bis 10 C-Atomen, Alkoxygruppen mit 1 bis 10 C-Atomen, Alkenyl- oder Alkinylgruppen mit 2 bis 10 C-Atomen, wie oben näher beschrieben, verstanden.

Es ist bevorzugt, dass X für S steht. Die zentrale Gruppe in Formel (I) ist dann eine Thienothiadiazol-Gruppe. Im Rahmen dieser Erfindung wird der Begriff "Thienothiadiazol" als Kurzform für "Thieno[3,4-c][1,2,5]thiadiazol" verwendet.

Für $Z^1$ ist es bevorzugt, dass es bei jedem Auftreten gleich oder verschieden für eine Einfachbindung, $-CR^3=CR^3-$ oder $-C{\equiv}C-$ steht. Besonders bevorzugt ist $Z^1$ eine Einfachbindung.

Für $Z^2$ ist es bevorzugt, dass es bei jedem Auftreten gleich oder verschieden für eine Einfachbindung, $-C(R^3)_2C(R^3)_2-$, $-CR^3=CR^3-$, $-C{\equiv}C-$, $-OC(R^3)_2-$ oder $-C(R^3)_2O-$ steht, besonders bevorzugt für eine Einfachbindung, $-CH_2CH_2-$, $-CF_2CF_2-$, $-CH=CH-$, $-CF=CF-$, $-C{\equiv}C-$, $-OCH_2-$, $-OCF_2-$, $-CH_2O-$ oder $-CF_2O-$. Besonders bevorzugt ist $Z^2$ eine Einfachbindung.

Für $Ar^1$ ist es bevorzugt, dass es bei jedem Auftreten gleich oder verschieden eine Arylgruppe mit 6 bis 15 C-Atomen oder eine Heteroarylgruppe mit 5 bis 15 C-Atomen darstellt, die mit einem oder mehreren Resten $R^4$ substituiert sein kann. Besonders bevorzugt ist $Ar^1$ bei jedem Auftreten gleich oder verschieden gewählt aus wahlweise mit Resten $R^4$ substituiertem Benzol, Fluoren, Naphthalin, Pyridin, Pyrimidin, Pyrazin, Triazin, Thiophen, Thiophen mit ankondensiertem 1,4-Dioxanring, Benzothiophen, Dibenzothiophen, Benzodithiophen, Cyclopentadithiophen, Thienothiophen, Indenothiophen, Dithienopyrrol, Silolodithiophen, Selenophen, Benzoselenophen, Dibenzoselenophen, Furan, Benzofuran, Dibenzofuran und Chinolin. Besonders bevorzugt ist Benzol oder Thiophen, die gegebenenfalls mit F substituiert sein können.

Die Gruppe $R^1$ ist bevorzugt bei jedem Auftreten gleich oder verschieden H, F, CN, $N(R^5)_2$, oder eine geradkettige Alkyl- oder Alkoxygruppe mit 1 bis 20 C-Atomen, die mit einem oder mehreren Resten $R^5$ substituiert sein kann, oder eine verzweigte Alkyl- oder Alkoxygruppe mit 3 bis 20 C-Atomen, die mit einem oder mehreren Resten $R^5$ substituiert sein kann, oder eine cyclische Alkylgruppe mit 4 bis 8 C-Atomen, die mit einem oder mehreren Resten $R^5$ substituiert

sein kann, wobei in den Alkyl- und Alkoxygruppen eine oder mehrere $CH_2$-Gruppen durch -O-, -S- oder -$R^5C=CR^5$- ersetzt sein können, oder eine Siloxanylgruppe mit 1 bis 10 Si-Atomen, die mit einem oder mehreren Resten $R^5$ substituiert sein kann.

Besonders bevorzugt ist $R^1$ bei jedem Auftreten gleich oder verschieden H, F, oder eine geradkettige Alkyl- oder Alkoxylgruppe mit 3 bis 20 C-Atomen, oder 3 bis 10 C-Atomen, die mit einem oder mehreren Resten $R^5$ substituiert sein kann, oder eine verzweigte Alkyl- oder Alkoxygruppe mit 3 bis 20 C-Atomen, die mit einem oder mehreren Resten $R^5$ substituiert sein kann, oder eine cyclische Alkylgruppe mit 6 C-Atomen, die mit einem oder mehreren Resten $R^5$ substituiert sein kann, wobei in den Alkyl- und Alkoxygruppen eine oder mehrere $CH_2$-Gruppen durch -O-, -S- oder -$R^5C=CR^5$- ersetzt sein können, oder eine Siloxanylgruppe mit 1 bis 6 Si-Atomen, die mit einem oder mehreren Resten $R^5$ substituiert sein kann, oder eine $N(R^5)_2$-Gruppe.

Ganz besonders bevorzugt ist $R^1$ ausgewählt aus einer geradkettige Alkyl- oder Alkoxylgruppe mit 3 bis 20, insbesondere 3 bis 10, C-Atomen, oder einer verzweigte Alkyl- oder Alkoxygruppe mit 3 bis 20, insbesondere 3 bis 10 C-Atomen, oder einer cyclische Alkylgruppe mit 6 C-Atomen, oder einer $N(R^{10})_2$-Gruppe, wobei $R^{10}$ Alkyl mit 1 bis 10 C Atomen ist.

In einer Ausführungsform weisen die Reste $R^1$ kein Fluor und/oder -CN auf. In einer weiteren Ausführungsform weisen sie keine -$CF_3$ Gruppe auf.

$R^3$ ist bevorzugt bei jedem Auftreten gleich oder verschieden H, F, oder eine Alkylgruppe mit 1 bis 10 C-Atomen, die mit einem oder mehreren Resten $R^5$ substituiert sein kann. Besonders bevorzugt ist $R^3$ bei jedem Auftreten gleich oder verschieden H oder F.

$R^4$ ist bevorzugt bei jedem Auftreten gleich oder verschieden H, D, F, CN, oder eine Alkyl- oder Alkoxygruppe mit 1 bis 10 C-Atomen, die mit einem oder mehreren Resten $R^5$ substituiert sein kann. Besonders bevorzugt ist $R^3$ bei jedem Auftreten gleich oder verschieden H oder F.

$R^5$ ist bei jedem Auftreten gleich oder verschieden H, F, CN, oder eine Alkyl- oder Alkoxygruppe mit 1 bis 10 C-Atomen, die mit einem oder mehreren Resten $R^6$ substituiert sein kann, oder eine Aryl- oder Heteroarylgruppe mit 5 bis 30 aromatischen Ringatomen, die jeweils mit einem oder mehreren Resten $R^6$ substituiert sein kann, oder eine Siloxanylgruppe mit 1 bis 6 Si-Atomen, die mit einem oder mehreren Resten $R^6$ substituiert sein kann.

Für den Index i gilt, dass er bevorzugt gleich 1, 2, oder 3 ist, besonders bevorzugt gleich 1 oder 2, ganz besonders bevorzugt gleich 1.

Die Verbindungen der Formel (I) sind keine Polymere, d.h. sie wurden nicht mittels Polymerisation hergestellt. Sie unterscheiden sich dadurch maßgeblich von im Stand der Technik beschriebenen Thienothiadiazol-Derivaten für die Photo- und Elektrochemie, die im Allgemeinen Polymere sind.

Bevorzugt weisen die Verbindungen der Formel (I) insgesamt 3 bis 5 aromatische Ringstrukturen auf. Mit aromatischer Ringstruktur wird damit ein Ring, oder ein anelliertes Ringsystem bezeichnet, das bevorzugt 2 bis 4 anellierte Ringe aufweist. Bevorzugt weist die Verbindung der Formel (I) 5 Ringsysteme auf, nämlich eine zentrale Thienothiadiazol-Gruppe und jeweils 2 seitliche miteinander verbundene aromatische Ringe.

Bevorzugt weisen die Reste $R^1$ jeweils mindestens 2 C-Atome, insbesondere jeweils mindestens eine Alkylkette mit mindestens 2 C-Atomen auf.

Besonders bevorzugt weisen die Verbindungen der Formel (I) insgesamt 3 bis 5 aromatische Ringstrukturen auf, und die Reste $R^1$ weisen jeweils mindestens eine Alkylkette mit mindestens 2 C-Atomen auf.

In einer bevorzugten Ausführungsform ist die Verbindung der Formel (I) eine chirale Verbindung. Sie wird bevorzugt als Racemat oder als Gemisch von Stereoisomeren (*d*, *l* oder *meso*) eingesetzt. Bevorzugt weist die Verbindung der Formel (I) eine oder zwei verzweigte Seitenketten $R^1$ auf, die ein Chiralitätszentrum aufweist.

In einer bevorzugten Ausführungsform ist die Verbindung der Formel (I) asymmetrisch. Dabei sind die beiden Substituenten an der zentralen Einheit nicht identisch. Solche Verbindungen weisen oft besondere elektrochemische Eigenschaften auf, insbesondere besondere Fluoreszenzeigenschaften.

In einer bevorzugten Ausführungsform weist die Verbindung der Formel (I) bei <200°C Mesophasen auf.

Bevorzugte Ausführungsformen der Formel (I) sind die folgenden Formel (Ia) und (Ib):

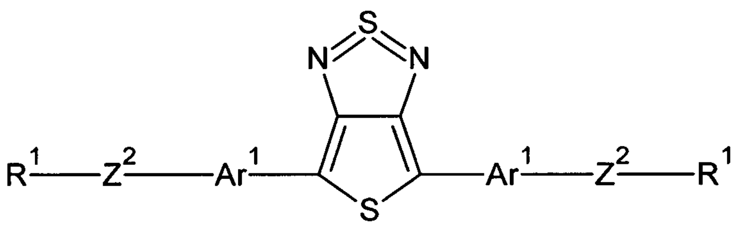

Formel (Ia)

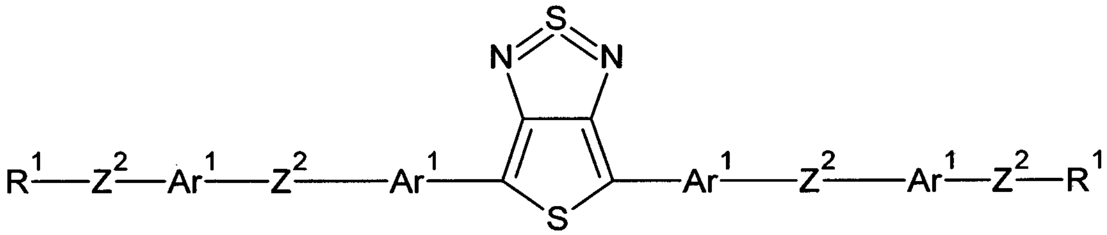

Formel (Ib)

wobei die auftretenden Gruppen wie oben definiert sind.

Bevorzugt gelten für Formeln (Ia) und (Ib) die oben angegebenen bevorzugten Ausführungsformen der Gruppen $Ar^1$, $Z^2$ und $R^1$.

Erfindungsgemäß und insbesondere für die Formeln (Ia) und (Ib) ist es bevorzugt, dass mindestens ein direkt an Thienothiadiazol gebundenes $Ar^1$ für eine schwefelhaltige Heteroarylgruppe steht, besonders bevorzugt für Thiophen. Die Gruppe kann mit einem oder mehreren Resten $R^4$ substituiert sein. Derartige Verbindungen zeichnen sich durch eine besonders hohe Lichtstabilität aus.

In einer bevorzugten Ausführungsform sind alle Reste $Z^1$ und $Z^2$ Einfachbindungen. Dann weist die Verbindung der Formel (I) die Formel (Ic) auf:

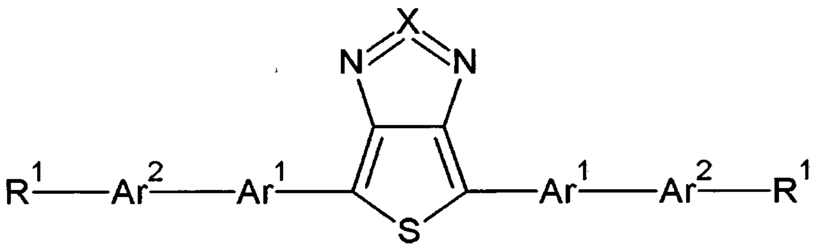

Formel (Ic)

wobei die auftretenden Gruppen wie oben definiert sind. Bevorzugt weisen die Formeln (Ic) und (Ib) die oben angegebenen bevorzugten Gruppen $Ar^1$ und $R^1$ auf, wobei $Ar^2$ ausgewählt ist wie $Ar^1$.

Bevorzugte Ausführungsformen von Verbindungen der Formel (Ia) und (Ib) sind die der folgenden Formeln (Ia-1) bis (Ia-4) und (Ib-1) bis (Ib-4):

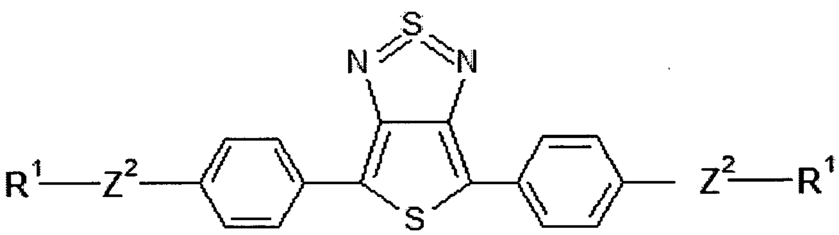

Formel (Ia-1)

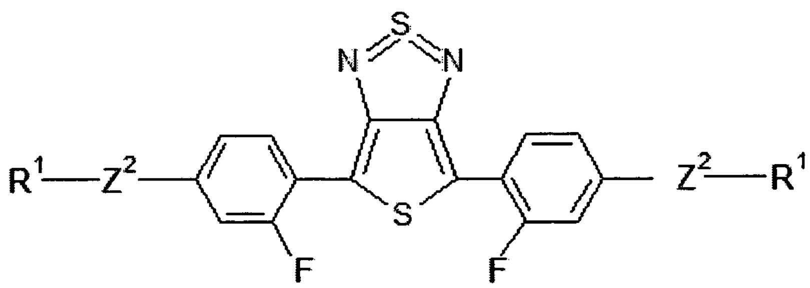

Formel (Ia-2)

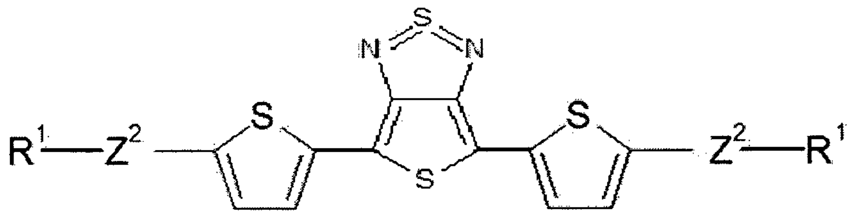

Formel (Ia-3)

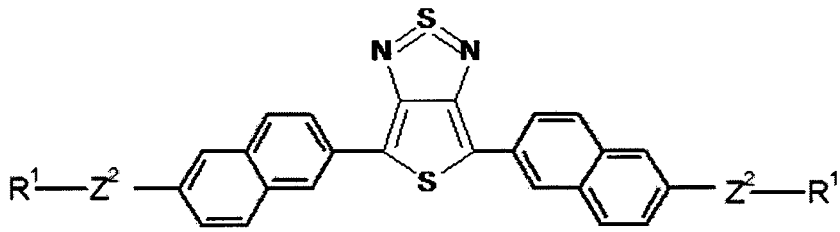

Formel (Ia-4)

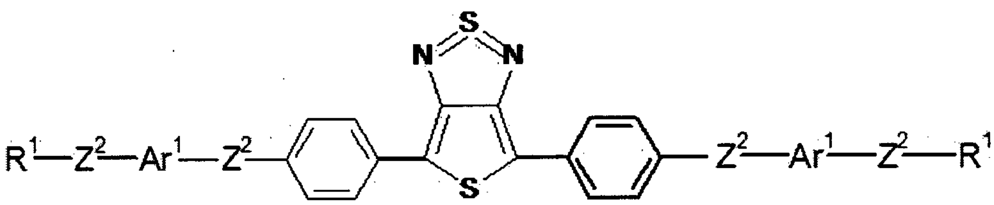

Formel (Ib-1)

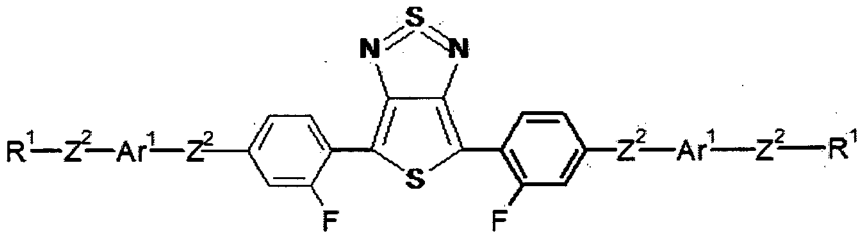

Formel (Ib-2)

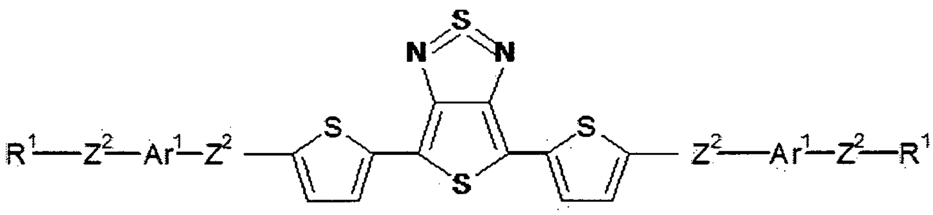

Formel (Ib-3)

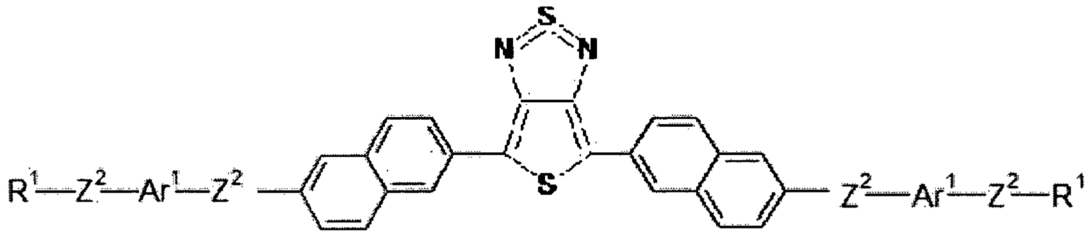

Formel (Ib-4),

wobei die auftretenden Gruppen $Ar^1$, $Z^2$ und $R^1$ definiert sind wie oben ausgeführt.

Besonders bevorzugte Verbindungen der Formel (I) sind die der folgenden Formel (IIa) bis (IIc):

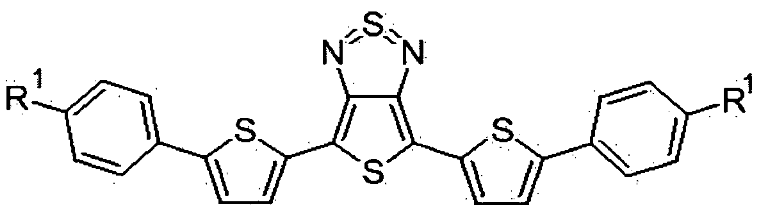

Formel (IIa)

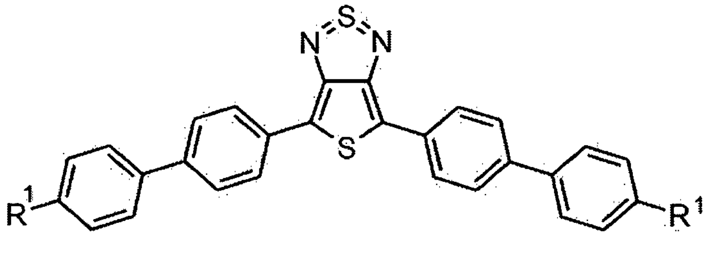

Formel (IIb)

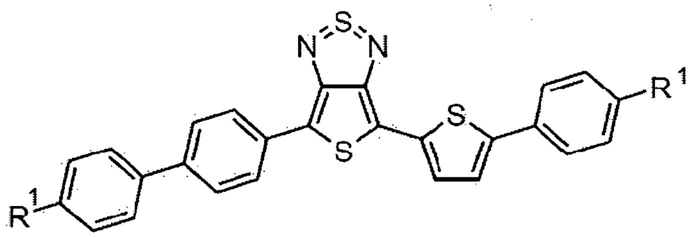

Formel (IIc)

wobei die Reste $R^1$ unabhängig voneinander definiert sind wie oben ausgeführt, und wobei die Benzol und Thiophenringe fluoriert sein können. Bevorzugt ist dabei nicht mehr als ein Fluor pro Ring vorhanden.

Weiterer Gegenstand der vorliegenden Erfindung sind Vorrichtungen, enthaltend spezielle Verbindungen gemäß Formel (I), die den folgenden Formeln (III) und (IV) entsprechen

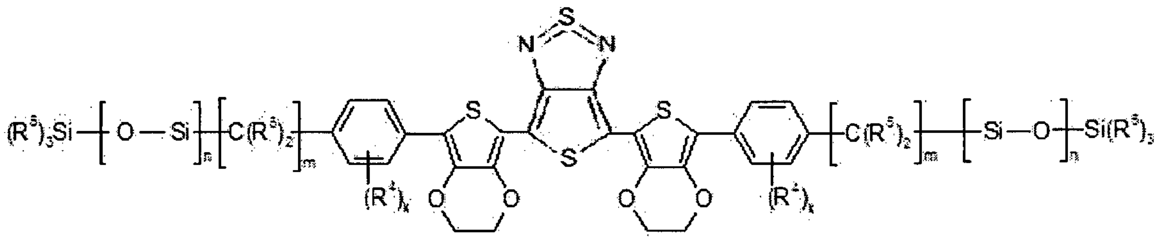

Formel (III)

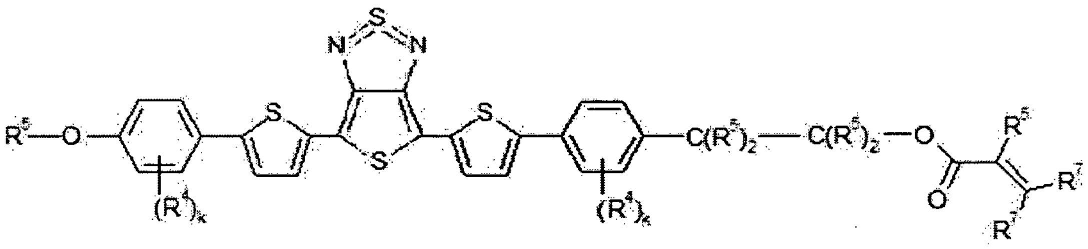

Formel (IV),

wobei gilt:

die auftretenden Gruppen $R^4$ und $R^5$ sind definiert wie oben, $R^7$ ist definiert wie $R^5$ oben, und

k ist, gleich oder verschieden bei jedem Auftreten, 0, 1, 2, 3 oder 4;

m ist, gleich oder verschieden bei jedem Auftreten, 0, 1, 2, 3, 4, 5 oder 6;

n ist, gleich oder verschieden bei jedem Auftreten, 1, 2, 3, 4 oder 5.

In einer bevorzugten Ausführungsform ist der Index k in den Formeln (III) oder (IV) gleich oder verschieden 0 oder 1, besonders bevorzugt gleich 0.

In einer bevorzugten Ausführungsform ist der Index m in den Formeln (III) oder (IV) gleich oder verschieden 0, 1 oder 2, besonders bevorzugt gleich 1 oder 2.

In einer bevorzugten Ausführungsform ist der Index n in den Formeln (III) oder (IV) gleich oder verschieden 1, 2 oder 3, besonders bevorzugt gleich 2.

Weiterhin bevorzugt ist $R^5$ in den Formeln (III) oder (IV) Wasserstoff oder eine Alkylgruppe mit 1 bis 5 C-Atomen, die mit einem oder mehreren Resten $R^6$ substituiert sein kann, besonders bevorzugt Methyl.

In einer bevorzugten Ausführungsform ist $R^7$ in der Formel (III) oder (IV) Wasserstoff oder eine Alkylgruppe mit 1 bis 5 C-Atomen die mit einem oder mehreren Resten $R^6$ substituiert sein kann, besonders bevorzugt Wasserstoff.

Die Verbindungen der Formel (III) und (IV) weisen besonders große Vorteile in Bezug auf die oben genannten

Eigenschaften gute Löslichkeit im flüssigkristallinen Medium, gute Lichtstabilität, hohe Fluoreszenz und/oder hohe Anisotropie der Absorption bzw. dichroitisches Verhältnis auf.

Die folgenden Verbindungen (1) bis (9) sind bevorzugte Verbindungen gemäß Formel (I):

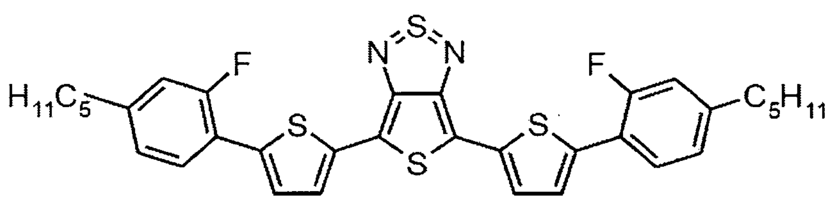

Verbindung (1)

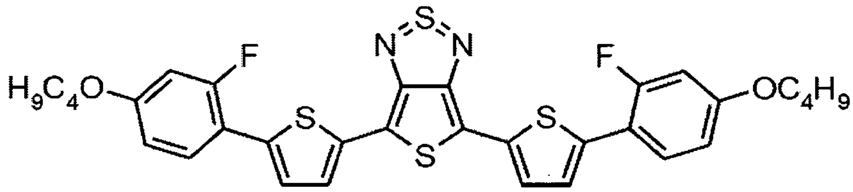

Verbindung (2)

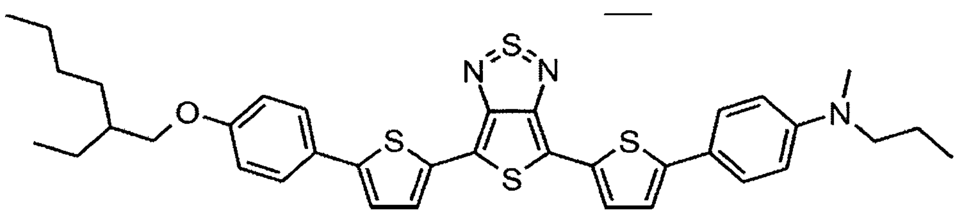

Verbindung (3)

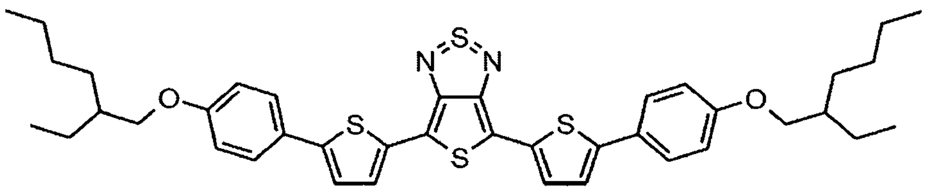

Verbindung (4)

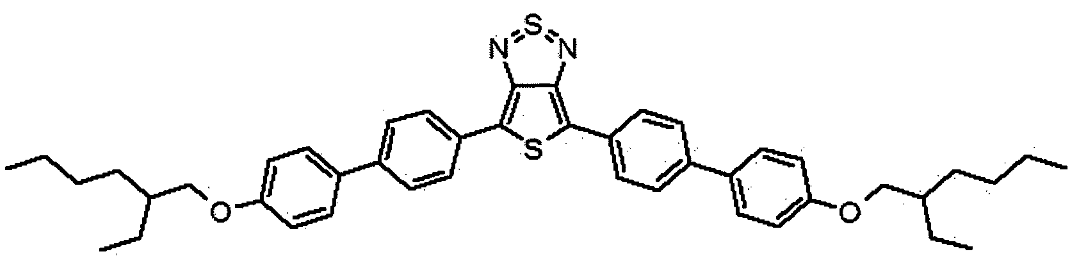

Verbindung (5)

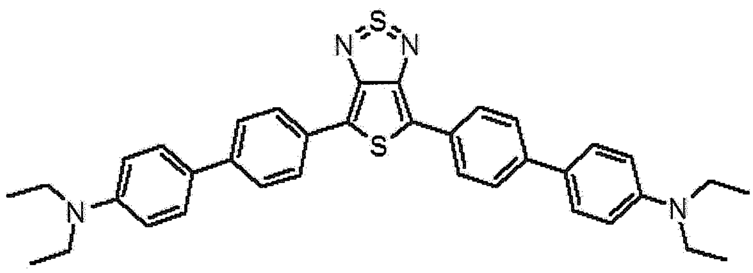

Verbindung (6)

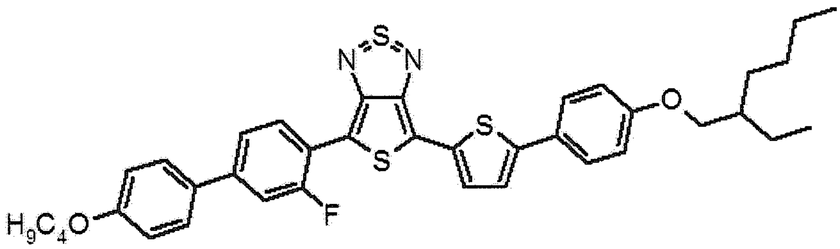

Verbindung (7)

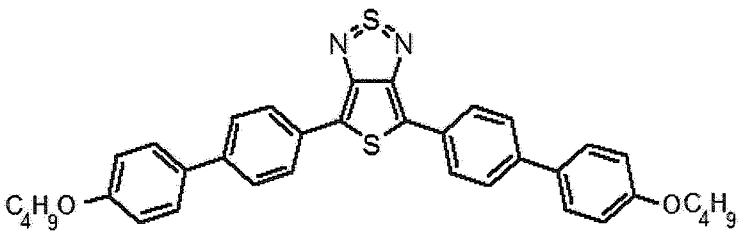

Verbindung (8)

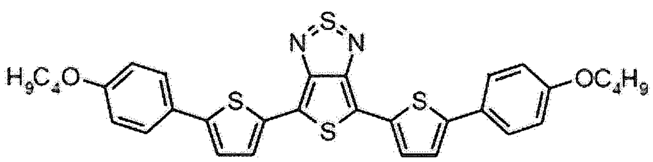

Verbindung (9)

Ein weiterer Gegenstand der Erfindung sind die in Anspruch 13 definierten Verbindungen der Formel (I):

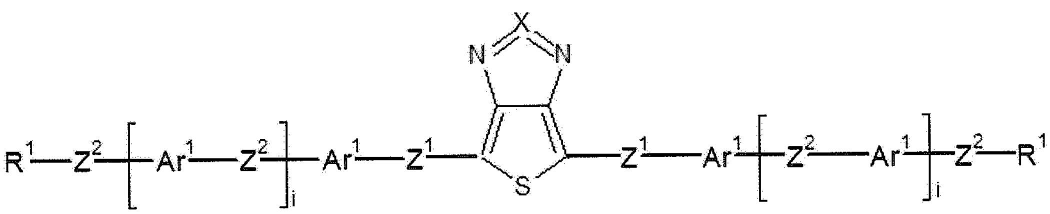

Formel (I),

wobei gilt:

X ist gleich S oder Se;

$Z^1$ ist unabhängig voneinander eine Einfachbindung, $-CR^3=CR^3-$ oder $-C\equiv C-$; oder zwei, drei, vier oder fünf miteinander kombinierte Gruppen, gewählt aus den Gruppen $-CR^3=CR^3-$ und $-C\equiv C-$;

$Z^2$ ist unabhängig voneinander eine Einfachbindung, O, S, $C(R^3)_2$, $-CR^3=CR^3-$ oder $-C\equiv C-$; oder zwei, drei, vier oder fünf miteinander kombinierte Gruppen, gewählt aus den Gruppen O, S, $C(R^3)_2$, $-CR^3=CR^3-$ und $-C\equiv C-$;

$Ar^1$ ist unabhängig voneinander eine Aryl- oder Heteroarylgruppe mit 5 bis 30 aromatischen Ringatomen, die mit einem oder mehreren Resten $R^4$ substituiert sein kann;

$R^1$ ist unabhängig voneinander eine geradkettige Alkyl- oder Alkoxygruppe mit 3 bis 10 C-Atomen oder eine verzweigte Alkyl- oder Alkoxygruppe mit 3 bis 10 C-Atomen;

$R^3$, $R^4$ sind unabhängig voneinander H, D, F, Cl, CN, oder eine Alkyl-, Alkoxy-, oder Thioalkoxygruppe mit 1 bis 10 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C=CR^5-$, $-C\equiv C-$, C=O, C=S, -C(=O)O-, -OC(=O)-, $Si(R^5)_2$, $NR^5$, -O- oder -S- ersetzt sein können;

$R^5$ ist unabhängig voneinander H, D, F, Cl, CN, $N(R^6)_2$, eine Alkyl-, Alkoxy- oder Thioalkoxygruppe mit 1 bis 10 C-Atomen oder eine Alkenyl- oder Alkinylgruppe mit 2 bis 20 C-Atomen, wobei die oben genannten Gruppen jeweils mit einem oder mehreren Resten $R^6$ substituiert sein können und wobei eine oder mehrere $CH_2$-Gruppen in den oben genannten Gruppen durch $-R^6C=CR^6-$, $-C\equiv C-$, C=O, C=S, -C(=O)O-, -O(C=O)-, $Si(R^6)_2$, $NR^6$, -O- oder -S- ersetzt sein können, oder eine Aryl- oder Heteroarylgruppe mit 5 bis 30 aromatischen Ringatomen, die jeweils mit einem oder mehreren Resten $R^6$ substituiert sein kann;

$R^6$ ist unabhängig voneinander H, F oder ein aliphatischer organischer Rest mit 1 bis 20 C-Atomen, in dem ein oder mehrere H-Atome durch F ersetzt sein können, oder eine Aryl- oder Heteroarylgruppe mit 5 bis 20 C-Atomen, in der ein oder mehrere H-Atome durch F ersetzt werden können; und

i ist unabhängig voneinander gleich 0, 1, 2, 3, 4 oder 5.

Diese Verbindungen können in den erfindungsgemäßen Vorrichtungen eingesetzt werden und erfindungsgemäß verwendet werden. In besonderen Ausführungsformen sind die Verbindungen so ausgewählt wie weiter oben im Zusammenhang mit der Vorrichtung ausgeführt. Dabei weisen die Verbindungen insbesondere eine Struktur auf gemäß Formel (Ia) oder (Ib), oder gemäß Formel (Ic), oder gemäß den Formeln (Ia-1) bis (Ia-4) und (Ib-1) bis (Ib-4), oder gemäß Formel (IIa), (IIb) oder (IIc). Besonders bevorzugt sind sie die Verbindungen (1), (2), (4), (5) und (7) bis (9) wie oben gezeigt.

Beschrieben hierin ist auch eine elektrochemische Vorrichtung, enthaltend mindestens eine der erfindungsgemäßen Verbindungen. Ferner beschrieben hierin ist auch die Verwendung einer solchen elektrochemischen Vorrichtung, oder der Verbindungen, in einem Fenster, insbesondere einem schaltbaren Fenster, in organischen Solarzellen, beispielsweise als Halbleiter (Donor oder Akzeptor) oder als Sensibilisator; in organischen Elektronikbauteilen, insbesondere Halbleitern, wie Feldeffekttransistoren, Dioden oder OLEDs, zum Anfärben einer Polymermatrix, beispielsweise im Automobilbereich; oder allgemein als Bestandteil flüssigkristalliner Mischungen gemäß der Erfindung, insbesondere zur Verwendung in einem Fenster.

Die oben beschriebenen Verbindungen der Formel (I) können gemäß grundsätzlich bekannten Verfahren der organischen Chemie hergestellt werden, insbesondere durch Suzuki-Kupplung zwischen organischen Bromiden und organischen Boronsäuren, oder durch Stille-Kopplung über die Tributylstannyl-Derivate. Im Folgenden werden besonders geeignete Verfahren in allgemeiner Form genannt. Für konkrete Verfahren zur Herstellung von Verbindungen der Formel (I) wird weiterhin auf die bekannte Literatur und auf die Ausführungsbeispiele verwiesen.

Ein mögliches, bevorzugtes Verfahren zur Herstellung von Verbindungen der Formel (I) basiert auf einem Thienothiadiazol-Derivat, welches zwei Brom- oder Chlor-Substituenten trägt. Durch Suzuki-Kupplung mit geeigneten Boronsäure-Derivaten können daraus Verbindungen der Formel (I) hergestellt werden, wie die folgenden Schemata 1 und 2 zeigen.

Schema 1

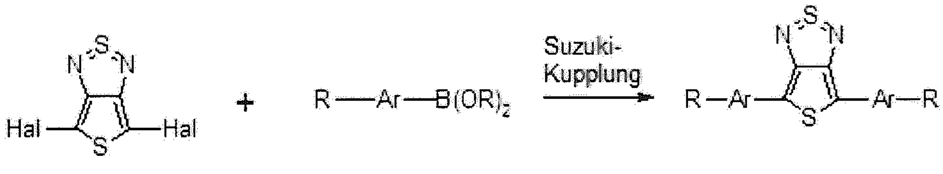


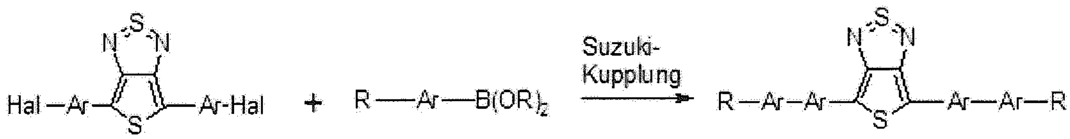


Die folgenden allgemeine Syntheseschemata 2 bis 5 zeigen beispielhaft, wie Verbindungen der Formel (I) herzustellen sind. Die Synthese geht vom Baustein 5 aus, dessen Herstellung in der Literatur beschrieben ist (Li et al., Dyes Pigm. 2011, 92, 674-680).

Schema 2:

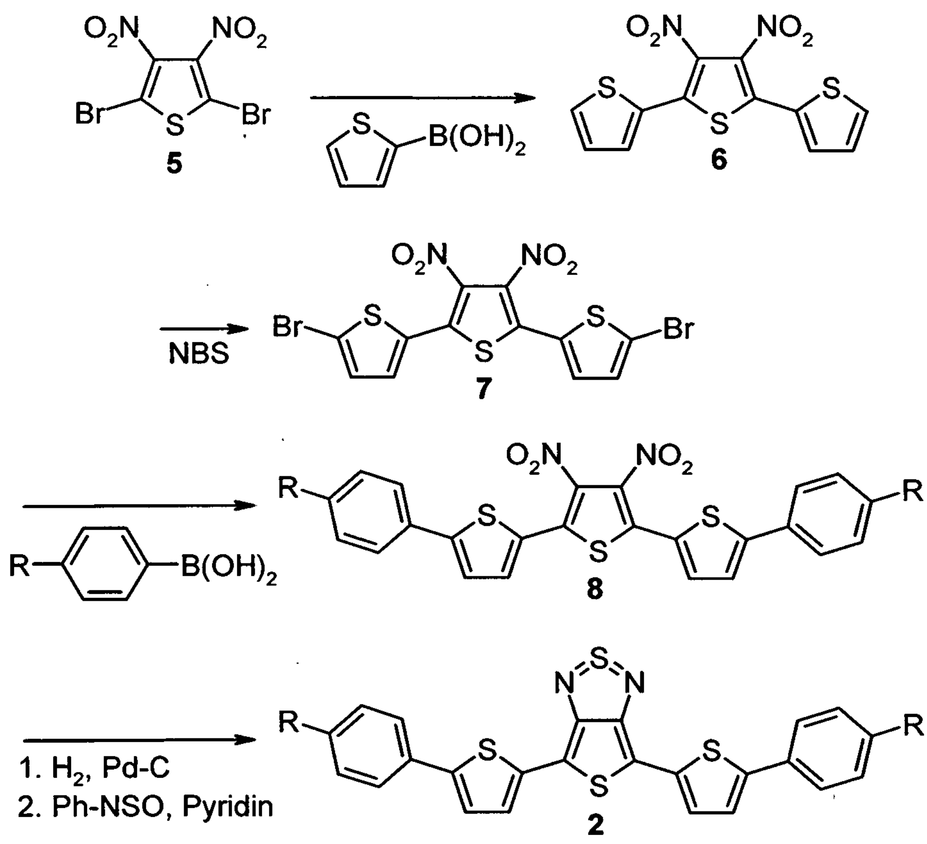

5
6
7
8
2

Schema 3:

8

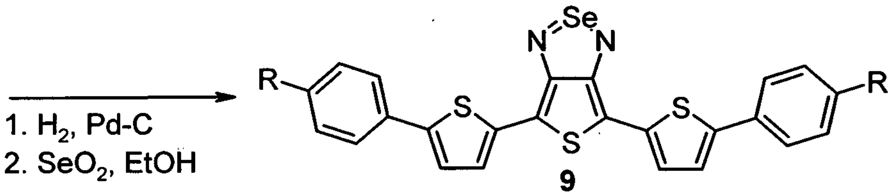


9

Schema 4:

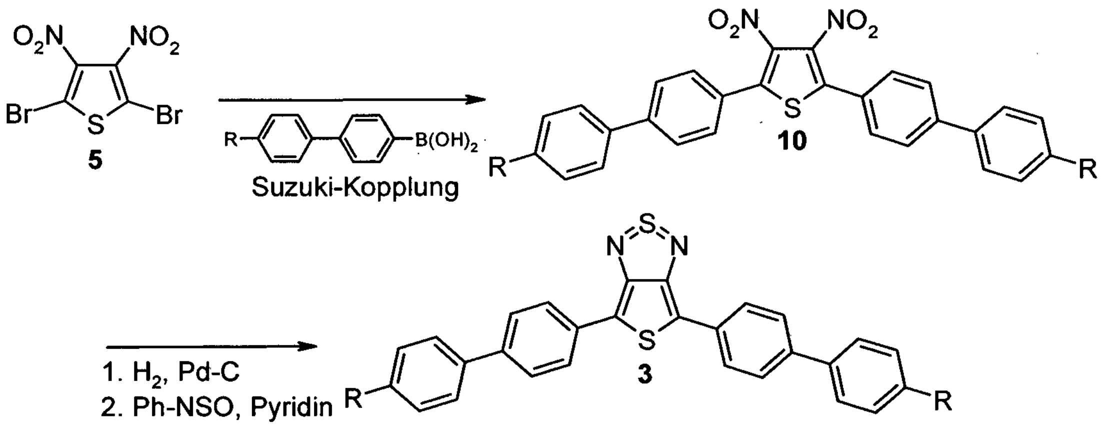


5

10

3

Schema 5:

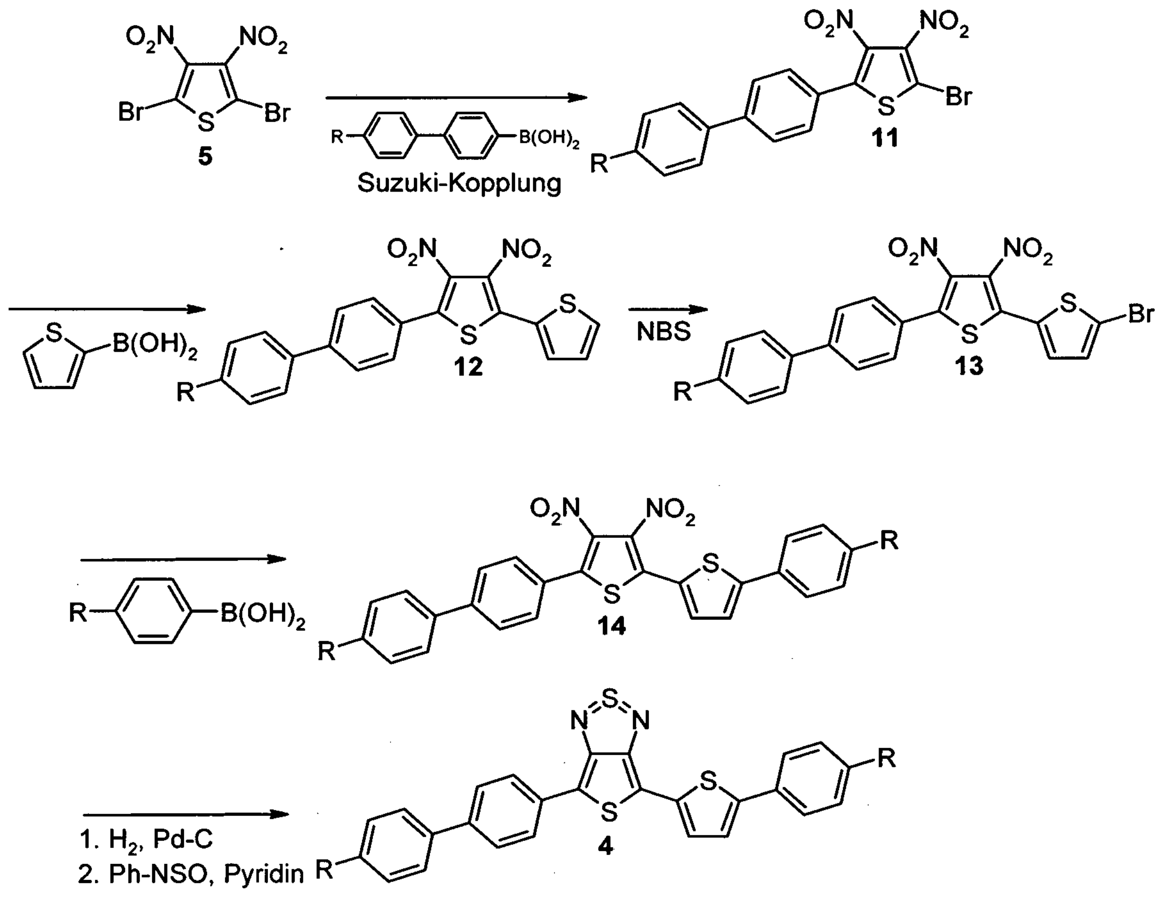


Synthesen von mit Aromaten substituierten Thienothiadiazolen werden auch in der WO2010/031479A1 und WO2013/102038 beschrieben. Auf die darin beschriebenen Syntheseverfahren wird hier ausdrücklich Bezug genommen wird.

Die Verbindung der Formel (I) ist bevorzugt ein positiv dichroitischer Farbstoff, d. h. ein Farbstoff, welcher einen positiven Anisotropiegrad R, ermittelt wie in den Ausführungsbeispielen angegeben, aufweist. Besonders bevorzugt ist der Anisotropiegrad R größer als 0.4, ganz besonders bevorzugt größer als 0.6 und am stärksten bevorzugt größer als 0.7, wobei R ermittelt wird wie in den Ausführungsbeispielen angegeben.

Bevorzugt erreicht die Absorption ein Maximum, wenn die Polarisationsrichtung des Lichts parallel zur Richtung der längsten Ausdehnung des Moleküls gemäß Formel (I) ist, und sie erreicht ein Minimum, wenn die Polarisationsrichtung des Lichts senkrecht zur Richtung der längsten Ausdehnung des Moleküls gemäß Formel (I) ist.

Die Verbindungen der Formel (I) weisen im allgemeinen eine gute Absorption oberhalb 580 nm auf. Bevorzugt weisen sie eine Absorptionsbande oberhalb 580 nm auf. Bevorzugt ist die längstwellige Absorption >580 nm.

Weiterhin bevorzugt ist die Verbindung der Formel (I) ein fluoreszierender Farbstoff. Unter Fluoreszenz wird dabei verstanden, dass eine Verbindung durch Absorption von Licht einer bestimmten Wellenlänge in einen elektronisch angeregten Zustand versetzt wird, wobei die Verbindung anschließend unter Emission von Licht in den Grundzustand übergeht. Bevorzugt hat das emittierte Licht längere Wellenlänge als das absorbierte Licht. Weiterhin bevorzugt ist der Übergang vom angeregten Zustand in den Grundzustand Spin-erlaubt, erfolgt also ohne Änderung des Spins. Weiterhin bevorzugt ist die Lebensdauer des angeregten Zustands der fluoreszierenden Verbindung kürzer als $10^{-5}$ s, besonders bevorzugt kürzer als $10^{-6}$ s, ganz besonders bevorzugt zwischen $10^{-9}$ und $10^{-7}$ s.

Die Verbindungen der Formel (I) weisen Vorteile gegenüber dem Stand der Technik auf und lösen dadurch die der Erfindung zugrunde liegende Aufgabe. Die Verbindungen zeigen in Lösung in einem flüssigkristallinen Host eine extreme Lichtechtheit, z. B. im Vergleich zu Perylendiimid-Derivaten. Außerdem zeigen sie eine langwellige Absorption, und hohen Dichroismus in Kombination mit sehr guter Löslichkeit. Ein besonderer Vorteil ist auch die Möglichkeit, über eine Variation der Elektron-Donor-Eigenschaften der Seitenketten, die Absorptions- und Emissionsmaxima auf ihre jeweiligen Anforderungen einzustellen.

Ein wesentlicher Vorteil ist die Lage des Fluoreszenz-Emissionsmaximums jenseits des Empfindlichkeitsbereichs des menschlichen Auges. In Anwendungen, wie beispielsweise schaltbaren Fenstern auf Basis flüssigkristalliner Guest-Host-Systeme, ermöglicht dies die Nutzung der emittierten Strahlung für die Elektrizitätsversorgung energieautarker Systeme mit Hilfe von Solarzellen, ohne dass das für das Auge unsichtbare Fluoreszenzlicht zu irritierenden Störeffekten bei solchen Fenstersystemen führt.

Die dichroitische Verbindung der Formel (I) liegt bevorzugt in einem Anteil von 0.01 bis 10 Gew.-%, besonders bevorzugt 0.05 bis 7 Gew.-% und ganz besonders bevorzugt 0.1 bis 7 Gew.-% in der Schaltschicht vor.

Neben der Verbindung der Formel (I) liegt in der Schaltschicht erfindungsgemäß ein flüssigkristallines Medium enthaltend eine oder mehrere verschiedene Verbindungen vor. Bevorzugt stellt das flüssigkristalline Medium die Hauptkomponente der Mischung der Schaltschicht der erfindungsgemäßen Vorrichtung dar. Die dichroitische Verbindung der Formel (I) liegt in der Schaltschicht bevorzugt in Lösung vor. Sie wird bevorzugt in ihrer Ausrichtung durch die Ausrichtung der Verbindungen des flüssigkristallinen Mediums beeinflusst.

Unter dem Begriff flüssigkristallines Medium wird im Rahmen der vorliegenden Erfindung ein Material verstanden, das unter bestimmten Bedingungen flüssigkristalline Eigenschaften aufweist. Bevorzugt hat das Material bei Raumtemperatur und in einer gewissen Temperaturspanne darüber und darunter flüssigkristalline Eigenschaften. Das flüssigkristalline Medium kann eine einzige Verbindung enthalten, oder es kann mehrere unterschiedliche Verbindungen enthalten. Typischerweise enthält das flüssigkristalline Medium gemäß der Erfindung mindestens eine Verbindung, deren Moleküle eine langgestreckte Form aufweisen, d.h. in einer Raumrichtung deutlich länger (Längsachse) sind als in den anderen beiden Raumrichtungen.

Weiterer Gegenstand der Erfindung ist die Verwendung einer Mischung, enthaltend ein flüssigkristallines Medium und mindestens eine Verbindung einer Formel (I), in einer Vorrichtung zur Regulierung des Energie-Durchtritts von einem Innenraum in einen Außenraum.

Bevorzugt hat das flüssigkristalline Medium der Schaltschicht einen Klärpunkt, bevorzugt einen Phasenübergang von einem nematisch flüssigkristallinen Zustand zu einem isotropen Zustand, im Temperaturbereich von 70 °C bis 170 °C, bevorzugt von 90 °C bis 160 °C, besonders bevorzugt von 95 °C bis 150 °C und ganz besonders bevorzugt von 105 °C bis 140 °C.

Bevorzugt ist weiterhin die dielektrische Anisotropie des flüssigkristallinen Mediums der Schaltschicht größer als 3, besonders bevorzugt größer als 7.

In einer weiteren bevorzugten Ausführungsform ist die dielektrische Anisotropie des flüssigkristallinen Mediums der Schaltschicht kleiner als null, besonders bevorzugt kleiner als -2.

Weiterhin bevorzugt hat das flüssigkristalline Medium der Schaltschicht eine optische Anisotropie ($\Delta n$) von 0.01 bis 0.3, besonders bevorzugt von 0.04 bis 0.27.

Weiterhin bevorzugt umfasst das flüssigkristalline Medium der Schaltschicht 3 bis 20 verschiedene flüssigkristalline Verbindungen, bevorzugt 8 bis 18, besonders bevorzugt 12 bis 16 verschiedene flüssigkristalline Verbindungen.

Verbindungen, welche als Bestandteile des flüssigkristallinen Mediums verwendet werden können, sind dem Fachmann bekannt und können beliebig gewählt werden.

Es ist bevorzugt, dass das flüssigkristalline Medium der Schaltschicht mindestens eine Verbindung enthält, welche Strukturelemente basierend auf 1,4-Phenylenen und 1,4-Cyclohexylenen aufweist, die mit einem oder mehreren Fluoratomen oder einer oder mehreren Nitrilgruppen substituiert sind. Besonders bevorzugt ist es, dass das flüssigkristalline Medium der Schaltschicht mindestens eine Verbindung enthält, welche 2, 3 oder 4, besonders bevorzugt 3 oder 4 Strukturelemente basierend auf 1,4-Phenylenen und 1,4-Cyclohexylenen aufweist.

Es ist weiterhin bevorzugt, dass das flüssigkristalline Medium der Schaltschicht einen oder mehrere chirale Dotierstoffe enthält. Bevorzugt liegen in diesem Fall in der Schaltschicht der Vorrichtung die Moleküle des flüssigkristallinen Medium gegeneinander verdrillt vor, besonders bevorzugt wie aus dem TN-Modus von Displays bekannt.

Chirale Dotierstoffe werden in dem flüssigkristallinen Medium der Schaltschicht bevorzugt in einer Gesamtkonzentration von 0.01 bis 3 Gew.-%, besonders bevorzugt von 0.05 bis 1 Gew.-% verwendet. Um hohe Werte für die Verdrillung zu erhalten, kann die Gesamtkonzentration der chiralen Dotierstoffe auch höher als 3 Gew.-% gewählt werden, bevorzugt bis maximal 10 Gew.-%.

Gemäß einer alternativen, ebenfalls bevorzugten Ausführungsform, enthält das flüssigkristalline Medium der Schaltschicht keine chiralen Dotierstoffe. Bevorzugt liegen in diesem Fall in der Schaltschicht der Vorrichtung die Moleküle des flüssigkristallinen Medium nicht gegeneinander verdrillt vor.

Die Anteile dieser Verbindungen und anderer Mindermengenkomponenten werden bei der Angabe der Anteile der flüssigkristallinen Verbindungen und der dichroitischen Farbstoffe vernachlässigt.

Das flüssigkristalline Medium der Schaltschicht enthält weiterhin bevorzugt einen oder mehrere Stabilisatoren. Die Gesamtkonzentration der Stabilisatoren liegt bevorzugt zwischen 0.00001 und 10 Gew.-%, besonders bevorzugt zwischen 0.0001 und 1 Gew.-% der Gesamtmischung. Die Anteile dieser Verbindungen und anderer Mindermengenkomponenten werden bei der Angabe der Anteile der flüssigkristallinen Verbindungen und der dichroitischen Farbstoffe vernachlässigt.

Die erfindungsgemäße Vorrichtung enthält bevorzugt in der Schaltschicht zusätzlich zu einer oder mehreren Verbindungen der Formel (I) und dem flüssigkristallinen Medium noch weitere dichroitische Farbstoffe mit anderer Struktur als Formel (I). Besonders bevorzugt enthält es einen, zwei, drei oder vier weitere Farbstoffe, ganz besonders bevorzugt zwei oder drei weitere Farbstoffe und am stärksten bevorzugt drei weitere Farbstoffe mit anderer Struktur als Formel (I).

Bezüglich der Eigenschaft des Dichroismus gelten die für die Verbindung der Formel (I) beschriebenen bevorzugten Eigenschaften auch für die optionalen weiteren dichroitischen Farbstoffe als bevorzugt.

Die Absorptionsspektren der dichroitischen Farbstoffe der Schaltschicht ergänzen sich bevorzugt derart, dass für das Auge der Eindruck von schwarzer Farbe entsteht. Bevorzugt decken die zwei oder mehr dichroitischen Farbstoffe des erfindungsgemäßen flüssigkristallinen Mediums einen großen Teil des sichtbaren Spektrums ab. Wie genau eine Mischung von Farbstoffen hergestellt werden kann, die für das Auge schwarz bzw. grau erscheint, ist dem Fachmann bekannt und beispielsweise in Manfred Richter, Einführung in die Farbmetrik, 2. Auflage, 1981, ISBN 3-11-008209-8, Verlag Walter de Gruyter & Co., beschrieben.

Die Einstellung des Farbortes einer Mischung aus Farbstoffen wird im Rahmen der Farbmetrik beschrieben. Hierzu werden die Spektren der Einzelfarbstoffe unter Berücksichtigung des Lambert-Beer'schen Gesetzes zu einem Gesamtspektrum berechnet und unter der zugehörigen Beleuchtung, z.B. für Tageslicht die Lichtart D65, gemäß den Regeln der Farbmetrik in die entsprechenden Farborte und Helligkeitswerte umgerechnet. Die Lage des Weißpunktes ist durch die jeweilige Lichtart, z.B. D65 festgelegt und in Tabellen (z. b. obenstehende Literaturstelle) aufgeführt. Durch Änderung der Anteile der verschiedenen Farbstoffe lassen sich verschiedene Farborte einstellen.

Gemäß einer bevorzugten Ausführungsform enthält die Schaltschicht zusätzlich zu der mindestens einen Verbindung der Formel (I) einen oder mehrere dichroitische Farbstoffe, die Licht im roten und NIR-Bereich, d.h. von 600 bis 2000 nm Wellenlänge absorbieren, bevorzugt im Bereich von 650 bis 1800 nm, besonders bevorzugt im Bereich von 650 bis 1300 nm. In einer bevorzugten Ausführung sind diese dichroitischen Farbstoffe gewählt aus Azoverbindungen, Anthrachinonen, Methinverbindungen, Azomethinverbindungen, Merocyaninverbindungen, Naphthochinonen, Tetrazinen, Perylenen, Terrylenen, Quaterrylenen, höheren Rylenen, Pyrromethenen, Azofarbstoffen, Nickeldithiolenen, (Metall-)Phthalocyaninen, (Metall-) Naphthalocyaninen und (Metall-)Porphyrinen. Besonders bevorzugt sind darunter Perylene und Terrylene.

Der Anteil aller dichroitischen Farbstoffe in der Mischung der Schaltschicht beträgt bevorzugt insgesamt 0.01 bis 10 Gew.-%, besonders bevorzugt 0.1 bis 7 Gew.-% und ganz besonders bevorzugt 0.2 bis 7 Gew.-%.

Die zusätzlichen, von Formel (I) unterschiedlichen, dichroitischen Farbstoffe der Schaltschicht sind bevorzugt ausgewählt aus den in B. Bahadur, Liquid Crystals - Applications and Uses, Vol. 3, 1992, World Scientific Publishing, Abschnitt 11.2.1 angegebenen Farbstoffklassen, und besonders bevorzugt aus den in der darin enthaltenen Tabelle aufgeführten expliziten Verbindungen.

Die genannten von Formel (I) unterschiedlichen Farbstoffe gehören zu den dem Fachmann bekannten Klassen von dichroitischen Farbstoffen, die vielfach in der Literatur beschrieben sind. So sind z.B. Anthrachinonfarbstoffe beschrieben in EP 34832, EP 44893, EP 48583, EP 54217, EP 56492, EP 59036, GB 2065158, GB 2065695, GB 2081736, GB 2082196, GB 2094822, GB 2094825, JP-OS 55-123673, DE 3017877, DE 3040102, DE 3115147, DE 3115762, DE 3150803 und DE 3201120, Naphthochinonfarbstoffe beschrieben in DE 3126108 und DE 3202761, Azofarbstoffe in EP 43904, DE 3123519, WO 82/2054, GB 2079770, JP-OS 56-57850, JP-OS 56-104984, US 4308161, US 4308162, US 4340973, T. Uchida, C. Shishido, H. Seki und M. Wada: Mol. Cryst. Lig. Cryst. 39, 39-52 (1977) und H. Seki, C. Shishido, S. Yasui und T. Uchida: Jpn. J. Appl. Phys. 21, 191-192 (1982), und Perylene beschrieben in EP 60895, EP 68427 und WO 82/1191. Rylen-Farbstoffe sind beispielsweise beschrieben in EP 2166040, US 2011/0042651, EP 68427, EP 47027, EP 60895, DE 3110960 und EP 698649.

Gemäß einer bevorzugten Ausführungsform enthält die Schaltschicht der erfindungsgemäßen Vorrichtung neben Verbindungen der Formel (I) ausschließlich dichroitische Farbstoffe, ausgewählt aus Rylen-Farbstoffen.

Beispiele für bevorzugte weitere von Formel (I) unterschiedlichen dichroitische Farbstoffe, die in der Schaltschicht der erfindungsgemäßen Vorrichtung enthalten sein können, sind in der folgenden Tabelle abgebildet:

| Tabelle 2 |
| --- |
| 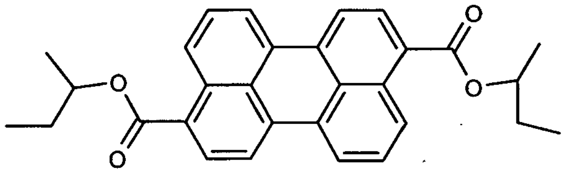 |
| |
| 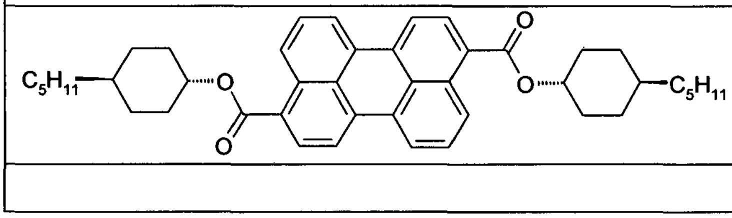 |
| |

| |
|---|
| 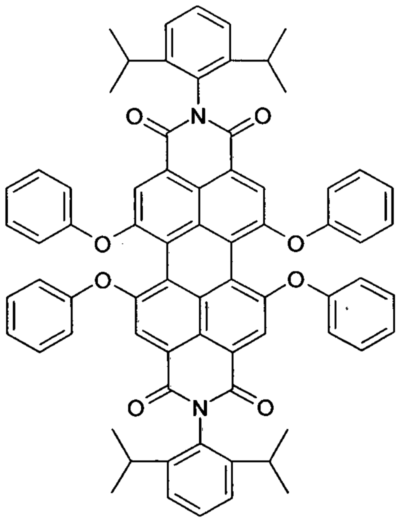 |
| |
| 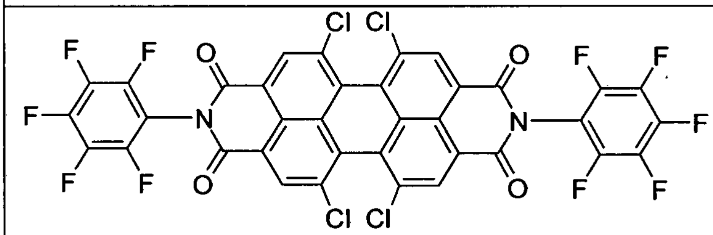 |
| |
| 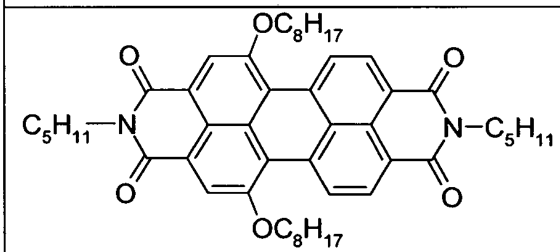 |
| |
| 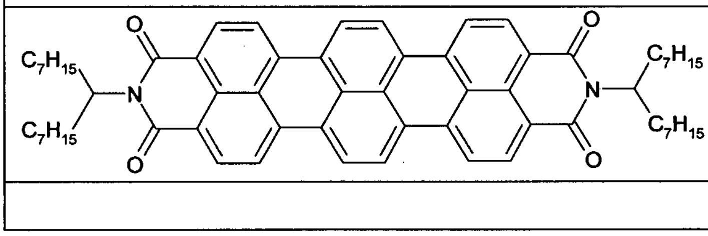 |
| |

| |
|---|
| 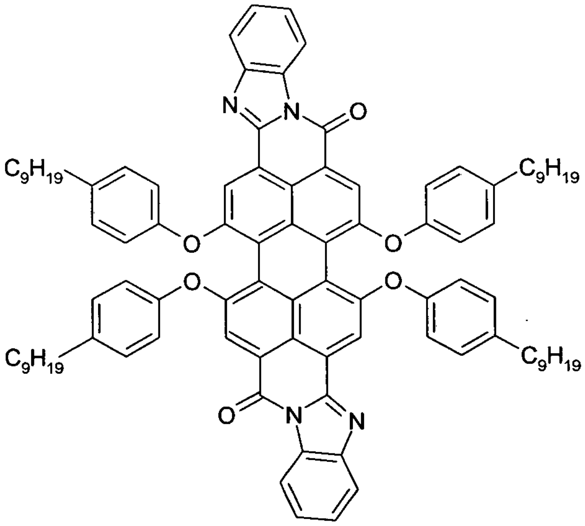 |
| |
| 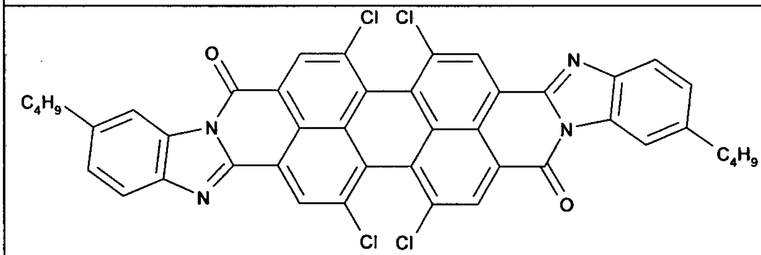 |
| |
| 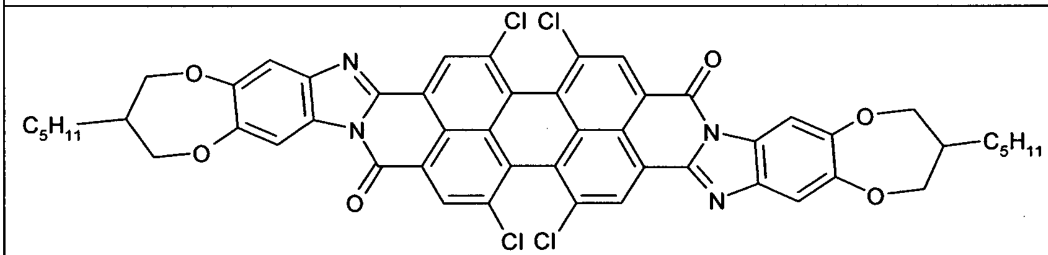 |
| |
| 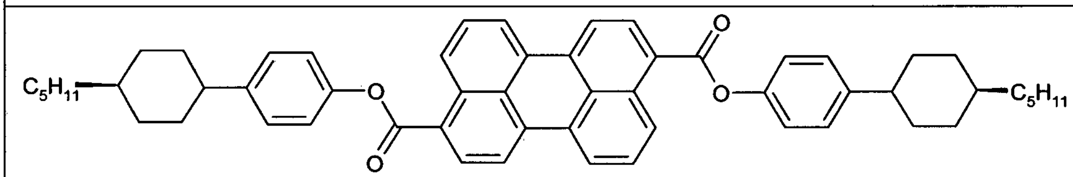 |
| |
| 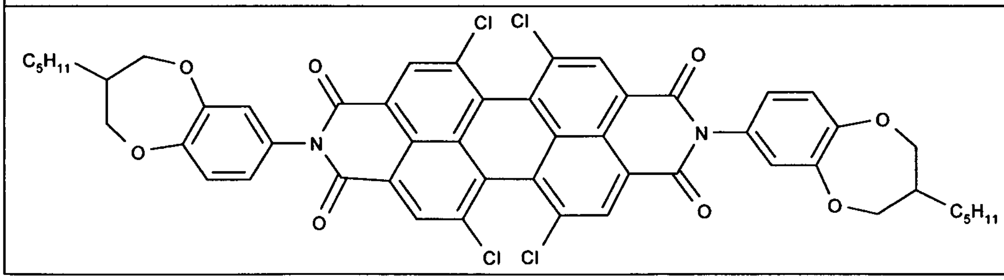 |

| |
|---|
| |
| 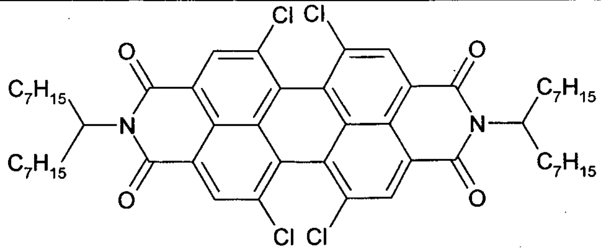 |
| |
| 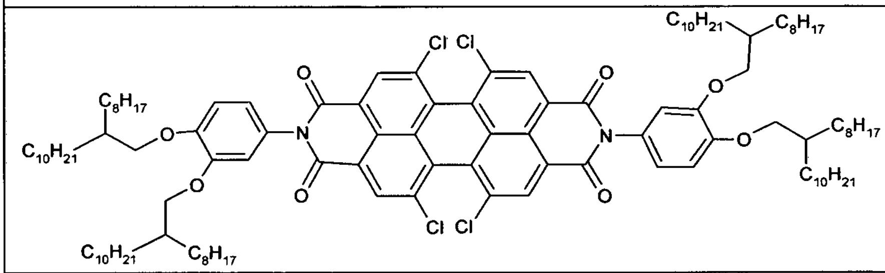 |
| |
| 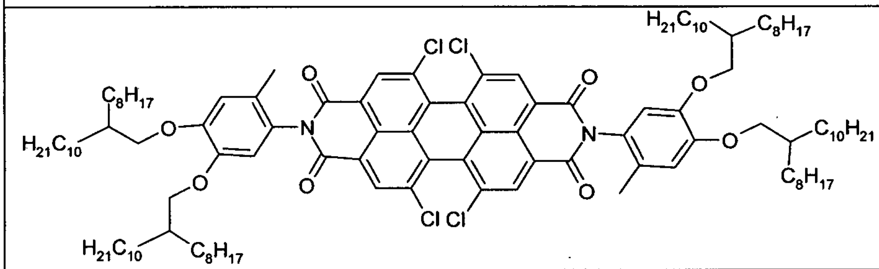 |
| |
| 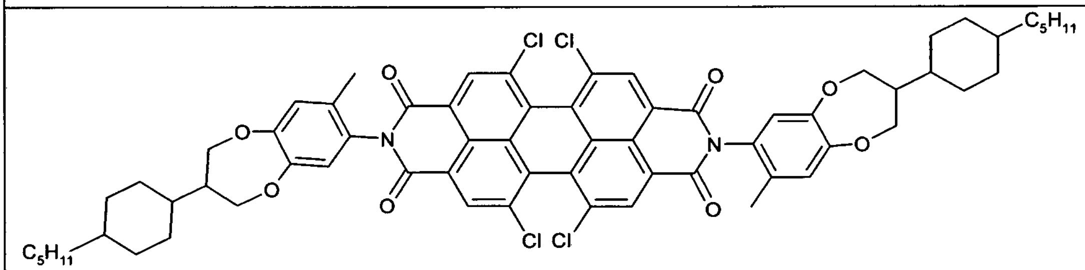 |
| |
| 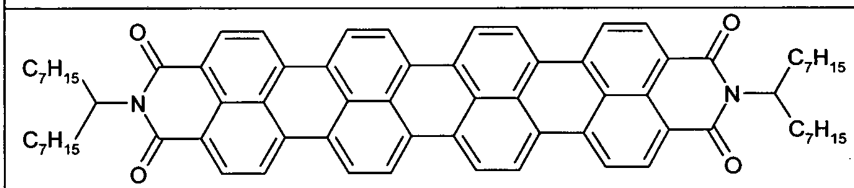 |
| |

| |
|---|
| 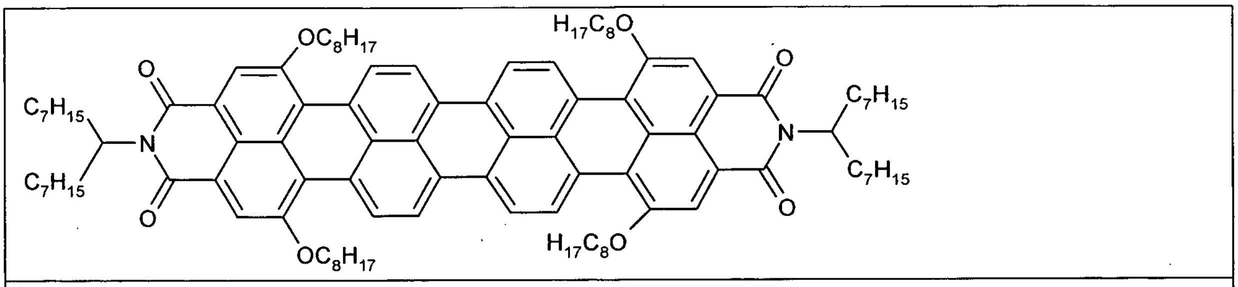 |
| |
| 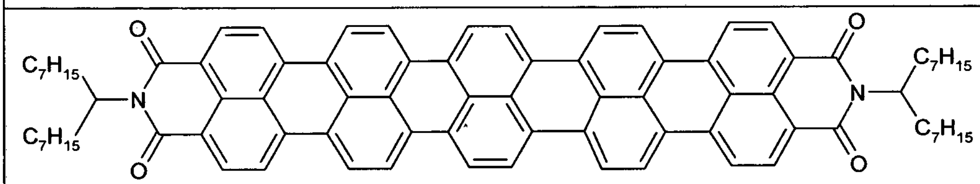 |
| |
| 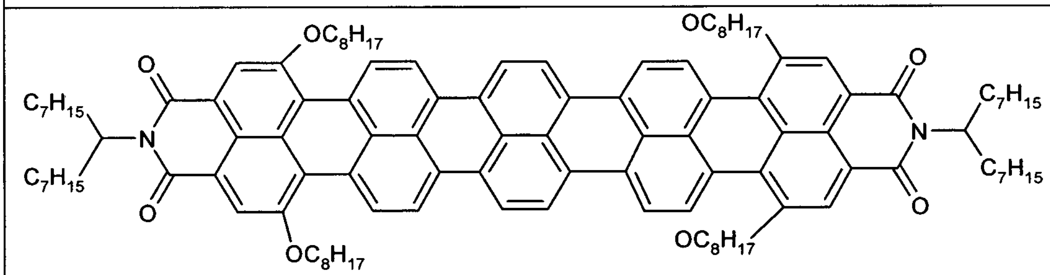 |
| |
| 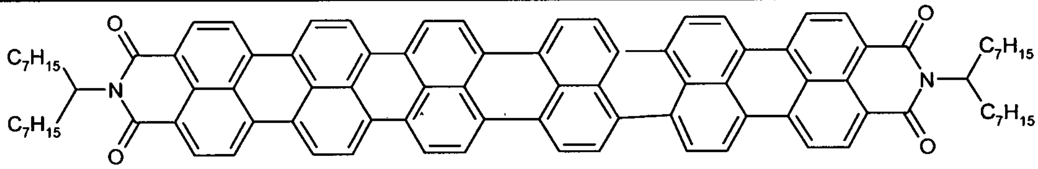 |
| |
| 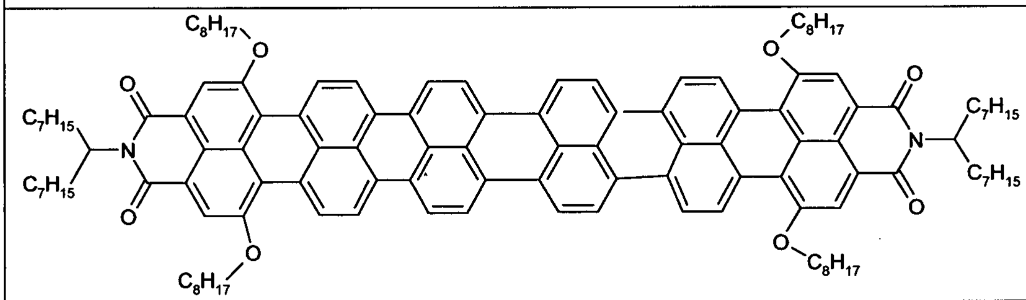 |
| |
| 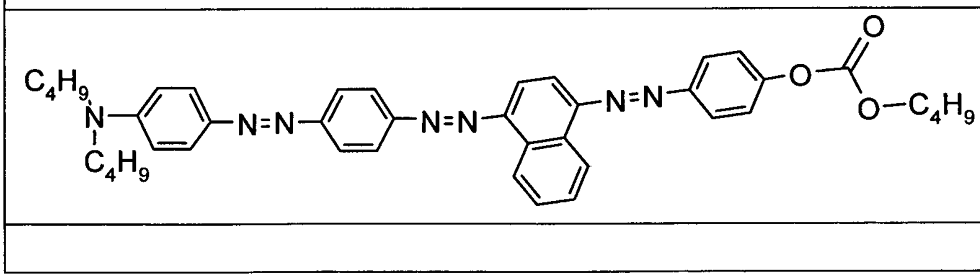 |
| |

| |
|---|
| 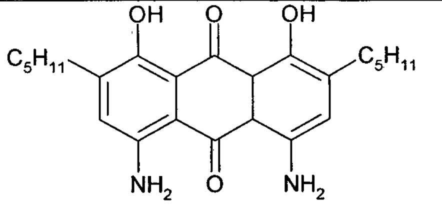 |
| |
| 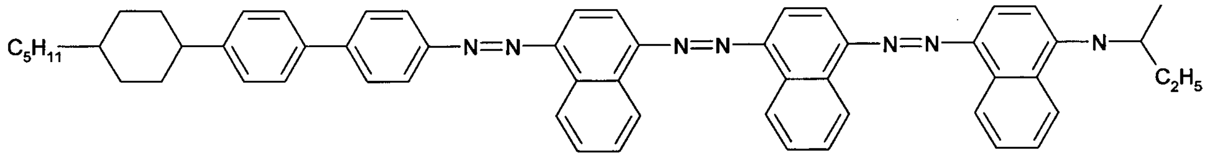 |
| |
| 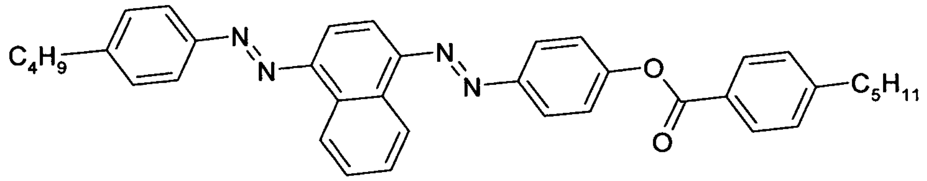 |
| |
| 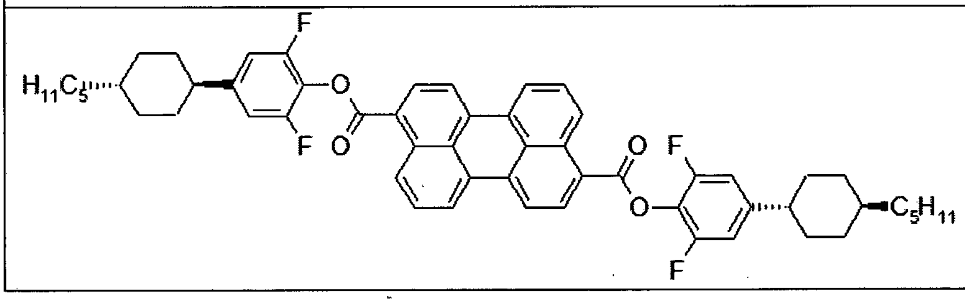 |

In einer bevorzugten Ausführungsform enthält die Schaltschicht der erfindungsgemäßen Vorrichtung eine oder mehrere Quencherverbindungen. Dies ist insbesondere dann bevorzugt, wenn die erfindungsgemäße Vorrichtung in ihrer Schaltschicht einen oder mehrere fluoreszierende Farbstoffe enthält.

Quencherverbindungen sind Verbindungen, welche die Fluoreszenz löschen. Die Quencherverbindungen können in der Schaltschicht die elektronische Anregungsenergie von Nachbarmolekülen, wie z.B. fluoreszierenden Farbstoffen, übernehmen und gehen dabei in einen elektronisch angeregten Zustand über. Der gequenchte fluoreszierende Farbstoff wechselt dadurch in den elektronischen Grundzustand und wird so an der Fluoreszenz oder einer Folgereaktion gehindert. Die Quencherverbindung selbst kehrt durch strahlungslose Desaktivierung oder durch Abgabe von Licht in den Grundzustand zurück und steht zum erneuten Quenchen wieder zur Verfügung.

In der Schaltschicht der erfindungsgemäßen Vorrichtung können der Quencherverbindung unterschiedliche Funktionen zukommen. Zum einen kann die Quencherverbindung zur Verlängerung der Lebensdauer eines Farbstoffsystems, durch Deaktivierung elektronischer Anregungsenergie, beitragen. Zum anderen eliminiert die Quencherverbindung ästhetisch ggfs. unerwünschte zusätzliche Farbeffekte, z.B. farbiges Abstrahlen in den Innenraum, die von den fluoreszierenden Farbstoffen in der Schaltschicht ausgehen.

Um ein effektives Quenchen zu erzielen, ist die Quencherverbindung dem jeweiligen Farbstoffsystem, insbesondere dem längstwelligst absorbierenden Farbstoff einer Farbstoffkombination, anzupassen. Wie dies zu tun ist, ist dem Fachmann bekannt.

Bevorzugte Quencherverbindungen sind beispielweise in Tabelle 8.1 auf Seite 279 in Joseph R. Lakowicz, Principles of Fluorescence Spectroscopy, 3rd Edition, 2010, ISBN 10: 0-387-31278-1, Verlag Springer Science+Business Media LLC, beschrieben. Weitere Molekülklassen sind beispielsweise unter den Stichworten Dark-Quencher oder Black Hole Quencher dem Fachmann geläufig. Beispiele sind Azofarbstoffe und Aminoanthrachinone. In der Schaltschicht der erfindungsgemäßen Vorrichtung können als Quencherverbindungen auch nicht-fluoreszierende oder nur im NIR fluoreszierende Farbstoffe zum Einsatz kommen.

In einer bevorzugten Ausführungsform der erfindungsgemäßen Schaltschicht sind gegebenenfalls enthaltene

Quencherverbindungen so gewählt, dass die Fluoreszenz im sichtbaren Teil des Spektrums unterdrückt wird.

Die erfindungsgemäße Vorrichtung ist bevorzugt zur Regulierung des Energie-Durchtritts in Form von von der Sonne ausgehendem Licht von der Umgebung in einen Innenraum geeignet. Dabei erfolgt der zu regulierende Energie-Durchtritt von der Umwelt (dem Außenraum) in einen Innenraum.

Der Innenraum kann dabei ein beliebiger weitgehend von der Umgebung abgeschlossener Raum sein, beispielsweise ein Gebäude, ein Fahrzeug, oder ein Container.

Gegenstand der Erfindung ist daher weiterhin die Verwendung der Vorrichtung zur Regulierung des Energie-Durchtritts von einem Außenraum in einen Innenraum.

Die Vorrichtung kann jedoch auch zur ästhetischen Raumgestaltung eingesetzt werden, beispielsweise für Licht- und Farbeffekte. Beispielsweise können Tür- und Wandelemente enthaltend die erfindungsgemäße Vorrichtung in grau oder in Farbe nach transparent geschaltet werden. Des Weiteren kann die Vorrichtung auch eine weiße oder farbige flächige Hinterleuchtung, die in der Helligkeit moduliert wird, oder eine gelbe flächige Hinterleuchtung, die mit einer blauen Gast-Wirtsanzeige in ihrer Farbe moduliert wird, umfassen. Es können eine oder beide Glasseiten der erfindungsgemäßen Vorrichtung mit angerauhtem oder strukturiertem Glas versehen sein zur Lichtauskopplung und/oder zur Lichteffekterzeugung.

In einer nochmals alternativen Verwendung wird die Vorrichtung zur Regulierung des Lichteinfalls auf die Augen eingesetzt, beispielsweise in Schutzbrillen, Visieren oder Sonnenbrillen, wobei die Vorrichtung in einem Schaltzustand den Lichteinfall auf die Augen gering hält, und in einem anderen Schaltzustand den Lichteinfall weniger stark verringert.

Die erfindungsgemäße Vorrichtung ist bevorzugt in einer Öffnung einer größeren flächigen Struktur angeordnet, wobei die flächige Struktur selbst nicht oder nur geringfügig Energie-Durchtritt zulässt, und wobei die Öffnung relativ gesehen energiedurchlässiger ist. Bevorzugt ist die flächige Struktur eine Wand oder eine sonstige Begrenzung eines Innenraums nach außen. Weiterhin bevorzugt bedeckt die flächige Struktur eine mindestens genauso große Fläche, besonders bevorzugt eine mindestens doppelt so große Fläche wie die Öffnung in ihr, in der die erfindungsgemäße Vorrichtung angeordnet ist.

Bevorzugt ist die Vorrichtung dadurch gekennzeichnet, dass sie eine Flächenausdehnung von mindestens 0.05 $m^2$ aufweist, bevorzugt mindestens 0.1 $m^2$, besonders bevorzugt mindestens 0.5 $m^2$ und ganz besonders bevorzugt mindestens 0.8 $m^2$.

Die Vorrichtung ist bevorzugt in einer wie oben beschriebenen, relativ gesehen energiedurchlässigeren Öffnung eines Gebäudes, eines Containers, eines Fahrzeugs oder eines sonstigen weitgehend geschlossenen Raums aufgebracht. Die Vorrichtung kann allgemein für beliebige Innenräume verwendet werden, besonders wenn diese nur begrenzten Luftaustausch mit der Umgebung aufweisen und lichtdurchlässige Begrenzungsflächen aufweisen, durch die Energie-Eintrag von außen in Form von Lichtenergie stattfinden kann. Besonders relevant ist die Verwendung der Vorrichtung für Innenräume, welche starker Sonneneinstrahlung durch lichtdurchlässige Flächen, beispielsweise durch Fensterflächen, ausgesetzt sind.

Die erfindungsgemäße Vorrichtung ist schaltbar. Unter Schaltung wird dabei eine Änderung des Energie-Durchtritts durch die Vorrichtung verstanden. Die erfindungsgemäße Vorrichtung ist bevorzugt elektrisch schaltbar, wie beispielsweise in WO 2009/141295 und in der Anmeldung EP 12008320.9 beschrieben wird.

Sie kann aber auch thermisch schaltbar sein, wie beispielsweise in WO 2010/118422 beschrieben. In diesem Fall erfolgt das Schalten bevorzugt durch einen Übergang von einem nematischen zu einem isotropen Zustand durch Änderung der Temperatur der Schaltschicht enthaltend die Verbindung der Formel (I) und das flüssigkristalline Medium. Im nematischen Zustand liegen die Moleküle des flüssigkristallinen Mediums geordnet vor und damit auch die Verbindung der Formel (I), beispielsweise ausgerichtet parallel zur Oberfläche der Vorrichtung durch die Wirkung einer Orientierungsschicht. Im isotropen Zustand liegen die Moleküle ungeordnet vor, und damit auch die Verbindung der Formel (I). Der Unterschied zwischen geordnetem und ungeordnetem Vorliegen der dichroitischen Verbindung der Formel (I) bewirkt einen Unterschied in der Lichtdurchlässigkeit der Schaltschicht der erfindungsgemäßen Vorrichtung, gemäß dem Prinzip, dass dichroitische Verbindungen je nach Orientierung in Bezug auf die Schwingungsebene des Lichts einen höheren oder einen niedrigeren Absorptionskoeffizienten aufweisen.

Ist die Vorrichtung elektrisch schaltbar, umfasst sie bevorzugt zwei oder mehr Elektroden, die zu beiden Seiten der Schaltschicht angebracht sind. Die Elektroden bestehen bevorzugt aus ITO oder aus einer dünnen, bevorzugt transparenten Metall- und/oder Metalloxidschicht, beispielsweise aus Silber oder FTO (Fluor-dotiertes Zinnoxid) oder einem alternativen, dem Fachmann für diese Verwendung bekannten Material. Die Elektroden sind bevorzugt mit elektrischen Anschlüssen versehen. Die Spannung wird bevorzugt durch eine Batterie, einen Akkumulator, oder durch externe Stromversorgung bereitgestellt.

Der Schaltvorgang erfolgt im Fall von elektrischer Schaltung durch eine Ausrichtung der Moleküle des flüssigkristallinen Mediums durch das Anlegen von Spannung.

In einer bevorzugten Ausführungsform wird dabei die Vorrichtung von einem Zustand mit hoher Absorption, d. h. geringer Lichtdurchlässigkeit, der ohne Spannung vorliegt, in einen Zustand mit geringerer Absorption, d. h. höherer Lichtdurchlässigkeit, überführt. Bevorzugt ist das flüssigkristalline Medium der Schaltschicht in beiden Zuständen nematisch. Der spannungslose Zustand ist bevorzugt dadurch gekennzeichnet, dass die Moleküle des flüssigkristallinen Mediums, und damit die Moleküle der Verbindung der Formel (I), parallel zur Ebene der Schaltschicht ausgerichtet vorliegen. Dies wird bevorzugt durch eine entsprechend gewählte Orientierungsschicht erreicht. Der Zustand unter Spannung ist bevorzugt dadurch gekennzeichnet, dass die Moleküle des flüssigkristallinen Mediums, und damit die Moleküle der Verbindung der Formel (I), senkrecht zur Ebene der Schaltschicht vorliegen.

In einer zur oben genannten Ausführungsform alternativen Ausführungsform wird die Vorrichtung von einem Zustand mit geringer Absorption, d. h. hoher Lichtdurchlässigkeit, der ohne Spannung vorliegt, in einen Zustand mit höherer Absorption, d. h. geringerer Lichtdurchlässigkeit, überführt. Bevorzugt ist das flüssigkristalline Medium der Schaltschicht in beiden Zuständen nematisch. Der spannungslose Zustand ist bevorzugt dadurch gekennzeichnet, dass die Moleküle des flüssigkristallinen Mediums der Schaltschicht, und damit die Moleküle der Verbindung der Formel (I), senkrecht zur Ebene der Schaltschicht ausgerichtet vorliegen. Dies wird bevorzugt durch eine entsprechend gewählte Orientierungsschicht erreicht. Der Zustand unter Spannung ist bevorzugt dadurch gekennzeichnet, dass die Moleküle des flüssigkristallinen Mediums der Schaltschicht, und damit die Moleküle der Verbindung der Formel (I), parallel zur Ebene der Schaltschicht vorliegen.

Gemäß einer bevorzugten Ausführungsform der Erfindung kann die Vorrichtung ohne externe Stromversorgung betrieben werden, indem die nötige Energie durch eine Solarzelle oder eine sonstige Vorrichtung zur Umwandlung von Licht und/oder Wärmeenergie in elektrische Energie bereitgestellt wird, die mit der Vorrichtung verbunden ist. Die Bereitstellung der Energie durch die Solarzelle kann direkt oder indirekt, d. h. über eine dazwischengeschaltete Batterie oder Akkumulator oder sonstige Einheit zur Speicherung von Energie, erfolgen. Bevorzugt ist die Solarzelle außen an der Vorrichtung angebracht oder sie ist innerer Bestandteil der Vorrichtung, wie beispielsweise in WO 2009/141295 offenbart. Bevorzugt sind dabei insbesondere Solarzellen, welche bei diffusem Licht besonders effizient sind, sowie transparente Solarzellen.

Die erfindungsgemäße Vorrichtung weist bevorzugt die folgende Schichtenabfolge auf, wobei zusätzlich weitere Schichten vorhanden sein können. Bevorzugt grenzen die im Folgenden angegebenen Schichten in der Vorrichtung direkt aneinander.

- Substratschicht, bevorzugt aus Glas oder Polymer
- elektrisch leitfähige transparente Schicht, bevorzugt aus ITO
- Orientierungsschicht
- Schaltschicht, enthaltend eine oder mehrere Verbindungen der Formel (I)
- Orientierungsschicht
- elektrisch leitfähige transparente Schicht, bevorzugt aus ITO
- Substratschicht, bevorzugt aus Glas oder Polymer

Die bevorzugten Ausführungsformen der einzelnen Schichten werden im Folgenden dargestellt.

Bevorzugt umfasst die erfindungsgemäße Vorrichtung eine oder mehrere, besonders bevorzugt zwei Orientierungsschichten. Die Orientierungsschichten liegen bevorzugt direkt angrenzend an die beiden Seiten der Schaltschicht gemäß Anspruch 1 enthaltend die Verbindung der Formel (I) vor.

Als Orientierungsschichten der erfindungsgemäßen Vorrichtung können beliebige dem Fachmann hierzu bekannte Schichten verwendet werden. Bevorzugt sind Polyimid-Schichten, besonders bevorzugt Schichten aus geriebenem Polyimid. Auf bestimmte, dem Fachmann bekannte Art geriebenes Polyimid führt zu einer Ausrichtung der Moleküle des flüssigkristallinen Mediums in Reiberichtung, wenn die Moleküle parallel zur Orientierungsschicht vorliegen (planare Ausrichtung). Dabei ist es bevorzugt, dass die Moleküle des flüssigkristallinen Mediums nicht völlig planar auf der Orientierungsschicht vorliegen, sondern einen leichten Aufstellwinkel aufweisen (pretilt). Zum Erreichen von vertikaler Ausrichtung der Verbindungen des flüssigkristallinen Mediums zur Oberfläche der Orientierungsschicht (homeotrope Ausrichtung) wird bevorzugt auf bestimmte Art behandeltes Polyimid als Material für die Orientierungsschicht eingesetzt (Polyimid für sehr hohe Pretiltwinkel). Weiterhin können Polymere, die durch einen Belichtungsvorgang mit polarisiertem Licht erhalten wurden, als Orientierungsschicht zum Erreichen einer Ausrichtung der Verbindungen des flüssigkristallinen Mediums gemäß einer Orientierungsachse verwendet werden (photoalignment).

Weiterhin bevorzugt ist in der erfindungsgemäßen Vorrichtung die Schaltschicht zwischen zwei Substratschichten angeordnet bzw. von diesen eingeschlossen. Die Substratschichten können beispielsweise aus Glas oder einem Polymer, bevorzugt einem lichtdurchlässigen Polymer, bestehen.

Bevorzugt ist die Vorrichtung dadurch gekennzeichnet, dass sie keinen Polarisator auf Polymerbasis, besonders bevorzugt keinen in fester Materiephase vorliegenden Polarisator und ganz besonders bevorzugt überhaupt keinen Polarisator umfasst.

Die Vorrichtung kann jedoch gemäß einer alternativen Ausführungsform auch einen oder mehrere Polarisatoren aufweisen. Bevorzugt sind die Polarisatoren in diesem Fall Linearpolarisatoren.

Wenn genau ein Polarisator vorhanden ist, so ist dessen Absorptionsrichtung bevorzugt senkrecht stehend

zur Orientierungsachse der Verbindungen des flüssigkristallinen Mediums der erfindungsgemäßen Vorrichtung auf derjenigen Seite der Schaltschicht, auf der sich der Polarisator befindet.

In der erfindungsgemäßen Vorrichtung können sowohl absorptive als auch reflektive Polarisatoren eingesetzt werden. Bevorzugt werden Polarisatoren verwendet, die als dünne optische Filme vorliegen. Beispiele für reflektive Polarisatoren, die in der erfindungsgemäßen Vorrichtung verwendet werden können, sind DRPF- (diffusive reflective polariser film, 3M), DBEF-(dual brightness enhanced film, 3M), DBR- (layered-polymer distributed Bragg reflectors, wie in US 7,038,745 und US 6,099,758 beschrieben) und APF-Filme (advanced polariser film, 3M, vgl. Technical Digest SID 2006, 45.1, US 2011/0043732 und US 7,023,602). Weiterhin können Polarisatoren basierend auf Drahtgittern (WGP, wire-grid-polarisers), welche Infrarotlicht reflektieren, eingesetzt werden. Beispiele für absorptive Polarisatoren, welche in den erfindungsgemäßen Vorrichtungen eingesetzt werden können, sind der Itos XP38-Polarisatorfilm und der Nitto Denko GU-1220DUN-Polarisatorfilm. Ein Beispiel für einen circularen Polarisator, welcher erfindungsgemäß verwendet werden kann, ist der Polarisator APNCP37-035-STD (American Polarizers). Ein weiteres Beispiel ist der Polarisator CP42 (ITOS).

Weiterhin bevorzugt enthält die erfindungsgemäße Vorrichtung ein Lichtleitsystem, das Licht zu einer Solarzelle oder einer sonstigen Vorrichtung zur Umwandlung von Licht und/oder Wärmeenergie in elektrische Energie leitet, bevorzugt wie in WO 2009/141295 beschrieben. Das Lichtleitsystem sammelt und konzentriert Licht, das auf die Vorrichtung trifft. Bevorzugt sammelt und konzentriert es Licht, das von fluoreszierenden dichroitischen Farbstoffen in der Schaltschicht emittiert wird. Das Lichtleitsystem steht mit einer Vorrichtung zur Umwandlung von Lichtenergie in elektrische Energie, bevorzugt einer Solarzelle, in Kontakt, so dass das gesammelte Licht konzentriert auf diese trifft. In einer bevorzugten Ausführungsform der Erfindung ist die Vorrichtung zur Umwandlung von Lichtenergie in elektrische Energie am Rand der Vorrichtung angebracht, in diese integriert und elektrisch mit Mitteln zur elektrischen Schaltung der erfindungsgemäßen Vorrichtung verbunden.

In einer bevorzugten Ausführungsform ist die erfindungsgemäße Vorrichtung Bestandteil eines Fensters, besonders bevorzugt eines Fensters enthaltend mindestens eine Glasfläche, ganz besonders bevorzugt eines Fensters, welche Mehrscheiben-Isolierglas enthält.

Unter Fenster wird dabei insbesondere eine Struktur in einem Gebäude verstanden, welche einen Rahmen und mindestens eine von diesem Rahmen umfasste Glasscheibe enthält. Bevorzugt enthält sie einen wärmeisolierenden Rahmen und zwei oder mehr Glasscheiben (Mehrscheiben-Isolierglas).

Gemäß einer bevorzugten Ausführungsform ist die erfindungsgemäße Vorrichtung direkt auf einer Glasfläche eines Fensters aufgebracht, besonders bevorzugt im Zwischenraum zwischen zwei Glasscheiben von Mehrscheiben-Isolierglas.

Ein Fenster enthaltend eine erfindungsgemäße Vorrichtung, bevorzugt mit den oben angegebenen bevorzugten Merkmalen, ist weiterer Gegenstand der Erfindung.

## Ausführungsbeispiele

Die folgenden Beispiele sollen die vorliegende Erfindung verdeutlichen und sind nicht beschränkend auszulegen.

In der vorliegenden Erfindung werden Strukturen von flüssigkristallinen Verbindungen durch Abkürzungen wiedergegeben (Akronyme). Diese Abkürzungen sind in WO 2012/052100 explizit vorgestellt und erklärt (S. 63 - 89).

Alle physikalischen Eigenschaften werden nach "Merck Liquid Crystals, Physical Properties of Liquid Crystals", Status Nov. 1997, Merck KGaA, Deutschland bestimmt und gelten für eine Temperatur von 20°C. Der Wert von $\Delta n$ wird bei 589 nm bestimmt und der Wert von $\Delta\varepsilon$ wird bei 1 kHz bestimmt, sofern nicht jeweils explizit anders angegeben. $n_e$ und $n_o$ sind jeweils die Brechungsindices des außerordentlichen und des ordentlichen Lichtstrahls unter den oben angegebenen Bedingungen.

Der Anisotropiegrad R wird ermittelt aus dem Wert für den Extinktionskoeffizienten E(p) (Extinktionskoeffizient der Mischung bei paralleler Ausrichtung der Moleküle zur Polarisationsrichtung des Lichts) und dem Wert für den Extinktionskoeffizienten der Mischung E(s) (Extinktionskoeffizient der Mischung bei senkrechter Ausrichtung der Moleküle zur Polarisationsrichtung des Lichts), jeweils bei der Wellenlänge des Maximums der Absorptionsbande des betreffenden Farbstoffs. Hat der Farbstoff mehrere Absorptionsbanden, so wird die intensivste Absorptionsbande ausgewählt. Die Ausrichtung der Moleküle der Mischung wird durch eine Orientierungsschicht erreicht, wie sie dem Fachmann auf dem Gebiet der LC-Displaytechnik bekannt ist. Zur Eliminierung von Einflüssen durch flüssigkristallines Medium, sonstigen Absorptionen und/oder Reflexionen wird jeweils gegen eine identisch vorliegende Mischung, die keinen Farbstoff enthält, gemessen und der erhaltene Wert subtrahiert.

Die Messung erfolgt mit linear polarisiertem Licht, dessen Schwingungsrichtung entweder parallel zur Orientierungsrichtung ist (Bestimmung von E(p)) oder senkrecht zur Orientierungsrichtung ist (Bestimmung von E(s)). Dies kann durch einen Linearpolarisator erreicht werden, wobei der Polarisator gegen die Vorrichtung gedreht wird, um die beiden unterschiedlichen Schwingungsrichtungen zu realisieren. Die Messung von E(p) und E(s) erfolgt also über die

Drehung der Schwingungsrichtung des eingestrahlten polarisierten Lichts.

Der Anisotropiegrad R wird aus den erhaltenen Werten für E(s) und E(p) nach der Formel

$$R=[E(p)-E(s)] / [E(p) + 2*E(s)]$$

berechnet, wie unter anderem in "Polarized Light in Optics and Spectroscopy", D. S. Kliger et al., Academic Press, 1990, angegeben. Eine detaillierte Beschreibung des Verfahrens zur Bestimmung des Anisotropiegrads von flüssigkristallinen Medien enthaltend einen dichroitischen Farbstoff findet sich auch in B. Bahadur, Liquid Crystals - Applications and Uses, Vol. 3, 1992, World Scientific Publishing, Abschnitt 11.4.2.

Die Größe $\varepsilon_{iso}$ wird wie folgt berechnet:

$\varepsilon_{iso} = [E(p) + 2*E(s)] / c / d$. Wobei die Konzentration c in % gewählt wird und die Schichtdicke d in cm.

**A) Herstellung der Farbstoffe**

Die Verbindung V-5 wird in einem Syntheseverfahren gemäß den folgenden Ausführungsbeispielen A-1 bis A-5 hergestellt.

A-1) Synthese der Verbindung V-1

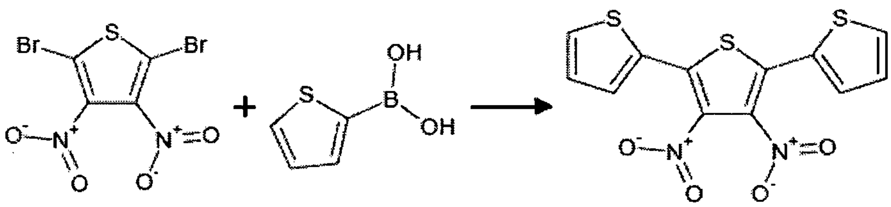

Das Metaborat wird in Wasser vorgelegt, Dibromid und THF, Katalysator sowie Hydrazin zugegeben und 30 sec. gerührt. Nun wird die Boronsäure zugegeben, auf Siedetemp. (65°C) erhitzt und über Nacht refluxiert. Gemäß Dünnschichtchromatogramm DC (Heptan/ EE 9:1) wird alles umgesetzt. Das Gemisch wird abgekühlt, mit Wasser und MTB-Ether versetzt und Phasen getrennt. Nun wird die wässrige Phase 6x mit MTB-Ether (Methyltert-butylether) extrahiert und die vereinigten org. Phasen 2x mit Wasser und 1x mit ges. NaCl-Lsg. gewaschen, über Natriumsulfat getrocknet. Es wird eine schlechte Phasentrennung (schwarz/schwarz) beobachtet. Danach wird zum Rückstand eingeengt. Der Rückstand wird mit Heptan/Toluol 1:1 über 200ml Kieselgel eluiert (Ausbeute: 6,3 g dunkelbraun, fest; HPLC: 52,2%). Das Produkt wird aus 40ml Toluol bei - 20°C kristallisiert (3,56g ockerbraunes Pulver; HPLC:91,7%).

Die folgenden Tabellen fassen die eingesetzten Ausgangsstoffe, das Produkt und die eingesetzten und erhaltenen Mengen zusammen.

| Name | Menge | M | Mol | Äquiv. |
|---|---|---|---|---|
| 2,5-Dibromo-3,4-dinitro-thiophen | 13.200 g | 331.928 | 39.768 mmol | 1.0 |
| Thiophen-2-boronsäure | 11.195 g | 127.958 | 87.489 mmol | 2.2 |
| Natriummetaborat-tetrahydrat | 21.929 g | 137.860 | 0.159 mol | 4.0 |
| Bis(triphenylphosphin)-palladium(II)-chlorid (15,2% Pd) zur Synthese | 1.117 g | 701.907 | 1.591 mmol | 0.0 |
| Tetrahydrofuran zur Analyse ACS | 300.000 ml | 72.106 | 3.703 mol | 93.1 |
| Hydraziniumhydroxid (etwa 80% $N_2H_5OH$) zur Synthese | 0.135 ml | 50.060 | 2.784 mmol | 0.1 |
| VE-Wasser | 120.000 g | 18.020 | 6.659 mol | 167.5 |

| Produkte | Menge | M | Gehalt | Mol | Ausbeute % | Th. Ausbeute |
|---|---|---|---|---|---|---|
| 3',4'-Dinitro-[2,2';5',2"]terthiophen | 3.560 g | 338.385 | 91.7% | 0.01 mol | 24.3 | 13.5 g |

A-2) Synthese der Verbindung V-2

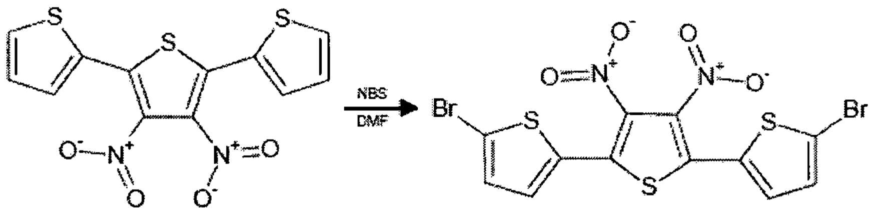


Die Verbindung V-1, wie gemäß Beispiel A-1) hergestellt, wird in DMF unter Stickstoff vorgelegt, NBS unter Rühren zugesetzt, das Reaktionsgemisch auf 90°C erhitzt und über Nacht bei dieser Temperatur gerührt. Die Reaktion wird mit einer Apparatur ausgeführt aus 100ml DHK, Magnetrührer, Kühler, Stickstoffbegasung, Thermometer und Heiztopf. Im DC ist die Herstellung des neuen Produkts erkennbar. Der Produkt-Ansatz wird noch im warmen mit Wasser versetzt. das Reaktionsgemisch färbt sich von rot zu dunkelbraun. Anschließend wird auf 0°C gekühlt und ausgefallene Kristalle abgesaugt. Als Rückstand wird 4,52 g orangebraunes Pulver erhalten (Verbindung V-2; HPLC: 82,3%).

Die folgenden Tabellen fassen die eingesetzten Ausgangsstoffe, das Produkt und die eingesetzten und erhaltenen Mengen zusammen.

| Name | Menge | M | Mol | Äquiv. |
|---|---|---|---|---|
| 3',4'-Dinitro-[2,2';5',2"]terthiophen | 3.500 g | 338.385 | 10.343 mmol | 1.0 |
| N-Bromsuccinimid zur Synthese | 3.866 g | 177.985 | 21.721 mmol | 2.1 |
| N,N-Dimethylformamid zur Analyse | 40.000 ml | 73.094 | 514.406 mmol | 49.7 |

| Produkt | Menge | M | Gehalt | Mol | Ausbeute % | Th. Ausbeute |
|---|---|---|---|---|---|---|
| 5,5"-Dibromo-3',4'-dinitro-[2,2';5', 2"]terthiophen | 4.520g | 496.177 | 82.3 % | 0.007 mol | 72.5 | 5.1 g |

A-3) Synthese der Verbindung V-3

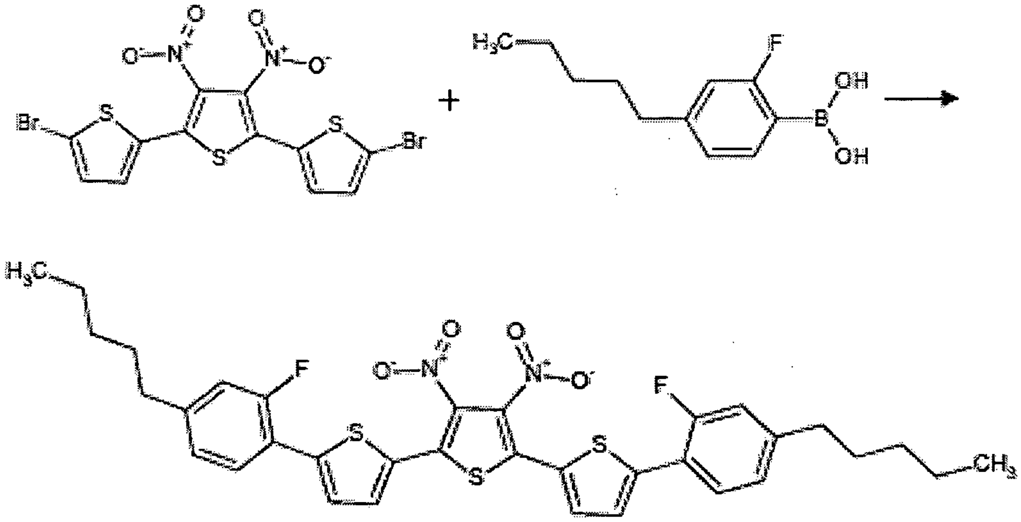

Das gemäß Beispiel A-2) hergestellte Dibromid V-2 und Boronsäure wird in 40 ml Toluol vorgelegt (unter Stickstoff). Die Carbonatlösung wird zum Ansatz zugegeben. Anschließend wird der Katalysator und der Ligand hinzugefügt, zum Sieden erhitzt und über Nacht unter Rückfluß gerührt. Gemäß Dünnschicht Kontrolle wird alles umgesetzt. Der Ansatz wird abkühlen gelassen und die wässrige Phase wird abgetrennt. Die wässrige Phase wird noch einmal mit Toluol extrahiert. Die vereinigten org. Phasen werden getrocknet, filtriert und einrotiert. Es wird 5,28g rotbraunes festes Rohprodukt erhalten. Es wird mit Toluol/Heptan 1:1 über eine 250 ml Kieselgelfritte eluiert. Die beiden dunkelroten Produktfraktionen werden vereinigt und eingeengt, und aus 35 ml Heptan/Toluol 4:1 kristallisiert. Das Produkt wird nicht vollständig gelöst(-20°C). Die Ausbeute beträgt 2,04g rote Flocken; HPLC:80,0%. Das Produkt wird aus 25ml Toluol bei 0°C kristallisiert. Ausbeute: 1,05g rote Flocken; HPLC:92,8%. Das Produkt wird erneut aus 15ml Toluol bei -20°C kristallisiert. Ausbeute: 0,88g rote Flocken; HPLC:97,3%.

Die folgenden Tabellen fassen die eingesetzten Ausgangsstoffe, das Produkt und die eingesetzten und erhaltenen Mengen zusammen.

| Name | Menge | Gehalt | M | Mol | Äquiv. |
|---|---|---|---|---|---|
| 5,5''-Dibromo-3',4'-dinitro-[2,2';5',2'']terthiophen | 2.000 g | 82.3 % | 496.177 | 3.317 mmol | 1.0 |
| G5-Boronsäure | 1.590 g | | 210.053 | 7.570 mmol | 2.3 |
| Tris(dibenzylidene-acetone)dipalladium (0) | 30.377 mg | | 915.700 | 0.033 mmol | 0.0 |
| Tris-(o-tolylphosphin) | 40.379 mg | | 304.300 | 0.133 mmol | 0.0 |
| Toluol reinst | 40.000 ml | | 92.140 | 377.686 | 113.9 |
| Natriumcarbonat (Lsg. 2 M) | 13.269 ml | 2.0 mol/l | 105.988 | 26.539 | 8.0 |

| Nr | **Produkte** | Menge | M | Gehalt | Mol | Ausbeute % | Th. Ausbeute |
|---|---|---|---|---|---|---|---|
| 1 | 5,5''-Bis-(2-fluoro-4-pentyl-phenyl)-3',4'-dinitro-[2,2';5',2''] terthiophen | 0.880 9 | 666.824 | 97.3 % | 0.001 mol | 38.7 | 2.2 g |

A-4) Synthese der Verbindung V-4

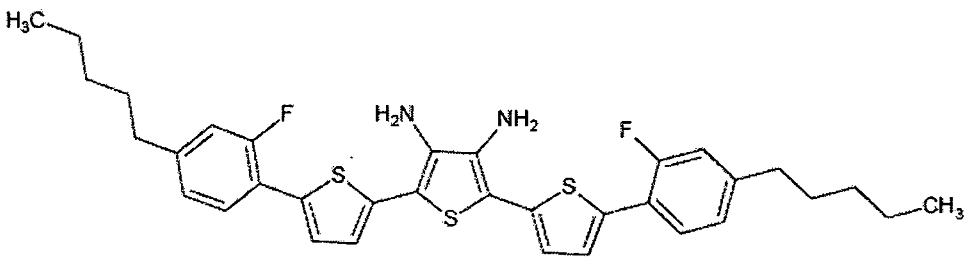

Verbindung V-4

Die Verbindung V-3, wie gemäß Beispiel A-3) hergestellt, wird zu der oben gezeigten Verbindung V-4 umgesetzt. Der Dehydrierungs-Katalysator und die weiteren Edukte sind in der Tabelle unten angegeben. Der Soll-Druck beträgt 4bar/RT, der Ist-Druck 4,4 bar/RT. Die Versuchsdauer ist 38 h und die Wasserstoffaufnahme 230 ml. Das Produkt wird abgesaugt und unter Argon abgefüllt. Die Hydrierlösung wird zum Rückstand einrotiert. Als Rückstand wird 1,27 g dunkles Produkt erhalten, HPLC: 42,0%, Umsatz +40,9% gemäß Dünnschichtchromatogramm.

Die folgenden Tabellen fassen die eingesetzten Ausgangsstoffe, das Produkt und die eingesetzten und erhaltenen Mengen zusammen.

| Name | Menge | Gehalt | M | Mol | Äquiv. |
|---|---|---|---|---|---|
| 5,5"-Bis-(2-fluoro-4-pentyl-phenyl)-3',4'-d initro-[2,2'; 5',2"]terthiophen | 0.900 g | 97.3 % | 666.824 | 0.001 mol | 1.0 |
| Tetrahydrofuran zur Analyse | 20.000 ml | | 72.106 | 212.299 mmol | 169.2 |
| Pd-C-5% E101 R (~54% H2O) Degussa | 0.200 g | | | | |
| Hydrogen/Wasserstoff 3.0 | 168.655 ml | | 22400.000 | 7.529 mmol | 6.0 |

| **Produkt** | Menge | M | Gehalt | Mol | Ausbeute % | Th. Ausbeute |
|---|---|---|---|---|---|---|
| 5,5"-Bis-(2-fluoro-4-pentyl-phenyl)-[2,2';5',2"]terthiophen e-3', 4'-diamin | 1.270 g | 606.858 | 42.0 % | 0.001 mol | 70.0 | 0.8 g |

A-5) Synthese der Verbindung V-5

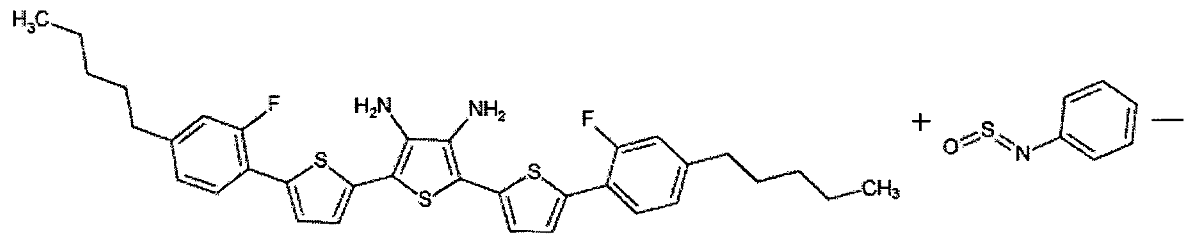

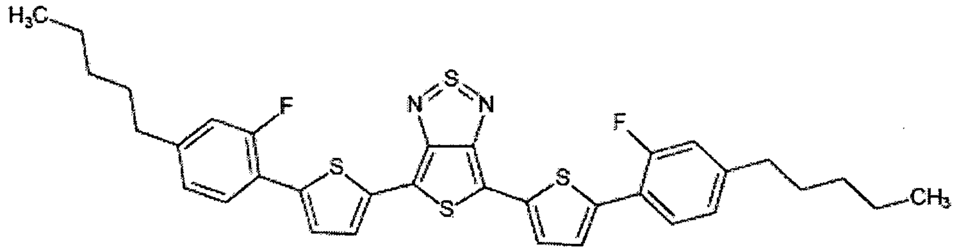

Die Verbindung V-4, wie gemäß Beispiel A-4) hergestellt, wird zu der oben gezeigten erfindungsgemäßen Verbindung V-5 umgesetzt.

Das Amin A-4) wird unter Argon im Kolben in Pyridin vorgelegt .Das N-Thionylanillin wird zugegeben und 5 h bei 50°C gerührt. Anschließend wird abgekühlt und bei Raumtemperatur übers Wochenende gerührt. Gemäß Dünnschichtchromatogramm (Toluol) erfolgt eine vollständige Umsetzung, ein neuer grüner Spot wird erhalten. Das Reaktionsgemisch wird mit MTB/Toluol versetzt und mehrmals mit HCl/Wasser gewaschen. Die organische Phase wird getrocknet und zum Rückstand einrotiert. Als Rohprodukt wird 1,3 g schwarzgrünes Öl erhalten. Das Rohprodukt wird mit Heptan/Chlorbutan 3:2 über eine Säule mit Kieselgel eluiert, und ein grüner Spot isoliert: 270 mg festes, dunkelgrünes Produkt; HPLC: 94,9%. Dieses wird aus Toluol 20ml kristallisiert bei -20°C. Als Produkt wird 222 mg blaugrün kohleartiges Pulver erhalten; HPLC: 96,2%. Die Strukturformel V-5 wird mittels Massenspektroskopie und NMR bestätigt.

Die folgenden Tabellen fassen die eingesetzten Ausgangsstoffe, das Produkt und die eingesetzten und erhaltenen Mengen zusammen.

| Name | Menge | Gehalt | M | Mol | Äquiv. |
|---|---|---|---|---|---|
| 5,5"-Bis-(2-fluoro-4-pentyl-phenyl)-[2,2';5 ',2"] terthiophene-3',4'-diamin | 1.270 g | 42.0% | 606.858 | 0.879 mmol | 1.0 |
| Pyridin zur Analyse | 10.000 ml | | 79.102 | 0.124 mol | 141.0 |
| N-Tionylanilin | 0.390 g | | 139.176 | 0.003 mol | 3.2 |

| **Produkte** | Menge | M | Gehalt | Mol | Ausbeute % | Th. Ausbeute |
|---|---|---|---|---|---|---|
| C34H32F2N2S4 | 220.000 mg | 634.892 | 96.2% | 0.000 mol | 38.3 | 558.0 mg |

<u>Beispiele A-6 bis A-8:</u>

Die Verbindung V-8 wird in einem Syntheseverfahren, wie in dem folgenden Schema gezeigt, gemäß den folgenden Ausführungsbeispielen A-6) bis A-8) hergestellt.

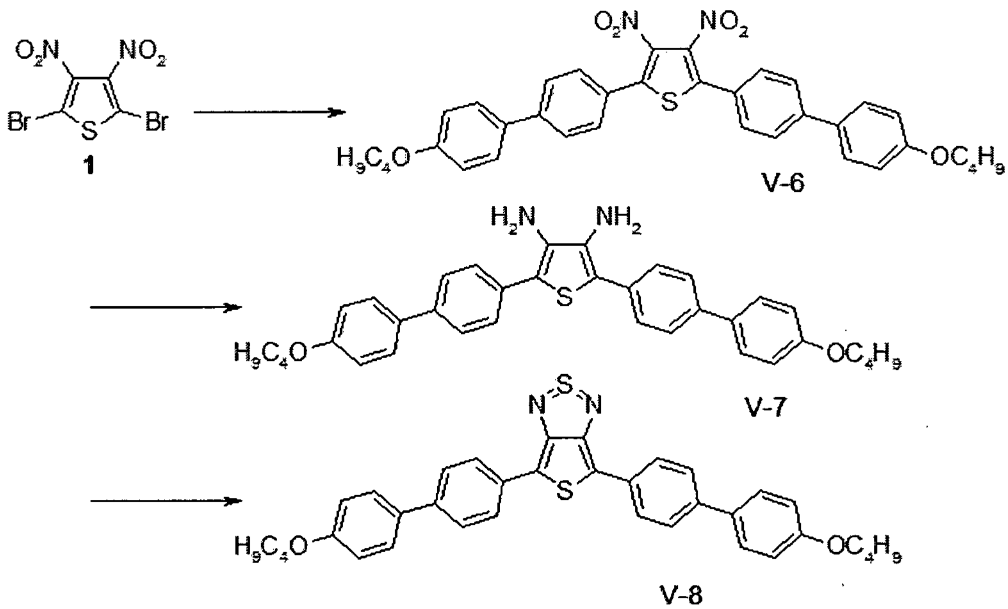

1

V-6

V-7

V-8

A-6) Synthese der Verbindung V-6

Eine Mischung aus Verbindung **1** (2 g, 6 mmol), 4-Butoxybiphenyl-4'-boronsäure (3.75 g, 13.3 mmol), Natriummetaborat-tetrahydrat (3.3 g, 24 mmol), Bis(triphenylphosphin)palladium(II)-Chlorid (169 mg, 0.24 mmol), THF (50 mL), Wasser (22.5 mL) und Hydrazinhydrat (0.02 mL, 0.42 mmol) wird 18 h lang unter Stickstoffatmosphäre bei 65°C gerührt. Die Mischung wird darauf wie üblich wäßrig aufgearbeitet, und man reinigt das Rohprodukt durch Filtration in Toluol über eine Kieselgelfritte (400 mL). Kristallisation aus Ethanol/Toluol 9:1 (40 mL) bei 0°C ergibt **V-6** als gelbe feine Nadeln (HPLC-Reinheit: 99.6%) in 16% Ausbeute.

A-7) Synthese der Verbindung V-7

Eine Lösung von Verbindung **V-6** (900 mg, 1 mmol) in THF (10 ml) wird in Gegenwart von 5% Pd-C (200 mg) bei 4 bar und RT bis zur Aufnahme der theoretischen Wasserstoffmenge hydriert. Der Katalysator wird abfiltriert und die Lösung zur Trockene einrotiert. Das Rohprodukt V-7 wird ohne weitere Aufreinigung in die nächste Synthesestufe eingesetzt.

A-8) Synthese der Verbindung V-8

Diamin **V-7** (800 mg, 1.42 mmol) wird unter Argonatmosphäre in Pyridin (10 mL) gelöst und unter Rühren mit *N*-Thionylanilin (396 mg, 3 mmol) versetzt. Man rührt für 17 h bei 50°C, arbeitet wie üblich wäßrig auf und rotiert die vereinigten organischen Extrakte zur Trockene ein. Das Rohprodukt (2.5 g blau-violett schimmernder Feststoff) wird in Toluol über eine Kieselgelfritte (300 mL) chromatographiert (540 mg, HPLC-Reinheit: 98.1%). Eine weitere Aufreinigung erfolgt durch Kristallisation aus Chlorbutan (50 mL) bei -20°C. Ausbeute: 466 mg (54%) **V-8** als dunkelblaue Kristalle.

A-9) Synthese der Verbindung V-9

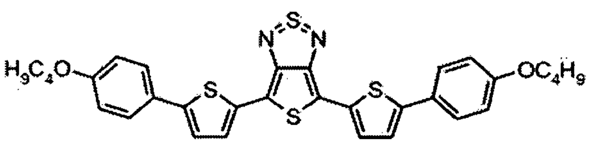

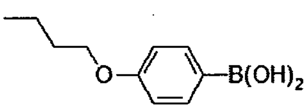

V-9

Boronsäure

Die Verbindung V-9) der oben gezeigten Formel wird analog zu den Ausführungsbeispielen A-1) bis A-5) hergestellt. Dabei wird Beispiel A-3) abgewandelt, indem die korrespondierende oben gezeigte Boronsäure eingesetzt wurde.

## B) Bestimmung der Eigenschaften der Farbstoffe

Die hergestellten Farbstoffe werden auf ihre physikalischen Eigenschaften hin untersucht, um ihre Eignung zur Verwendung in Vorrichtungen zur Regulierung der Energie-Transmission festzustellen. Zum Vergleich werden die entsprechenden Eigenschaften für die Verbindung D-1 (Struktur siehe unten) angegeben.

Tabelle: Strukturen der verwendeten Farbstoffe

| |
|---|
| 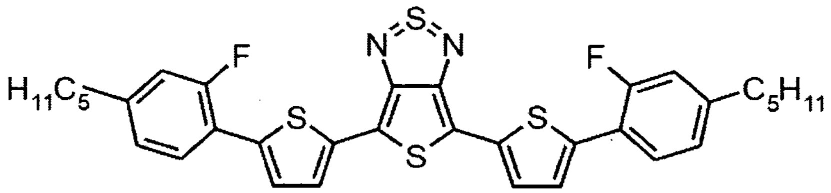 |
| Verbindung (1) |
| |
| 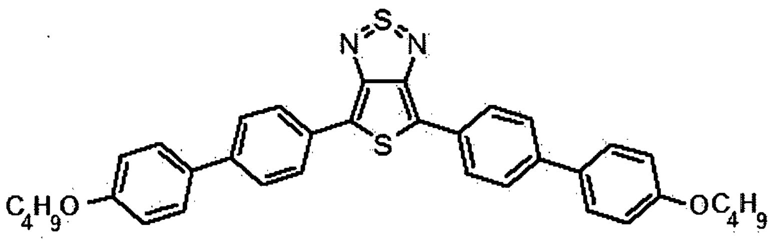 |
| Verbindung (8) |
| |
| 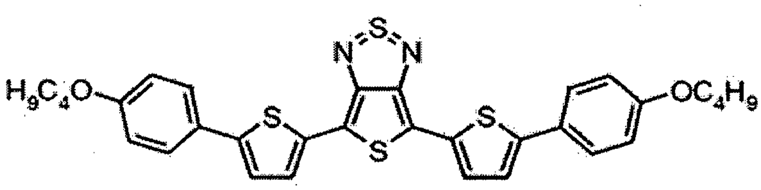 |
| Verbindung (9) |
| |
| 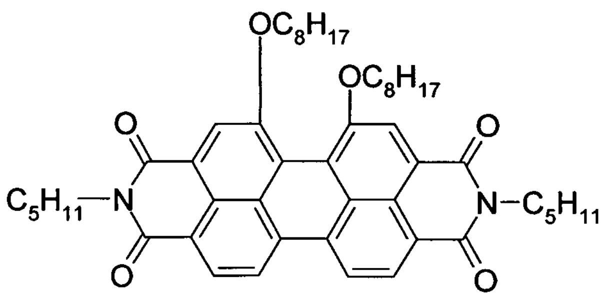 |
| D-1 |

(fortgesetzt)

| |
|---|
| 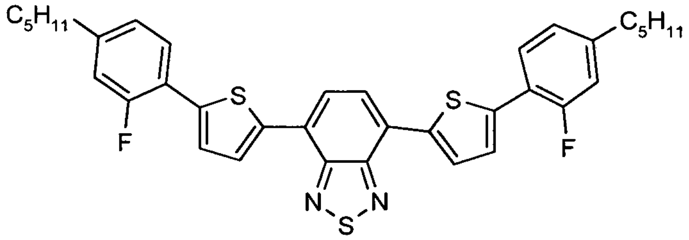 |
| D-2 |
| |
| 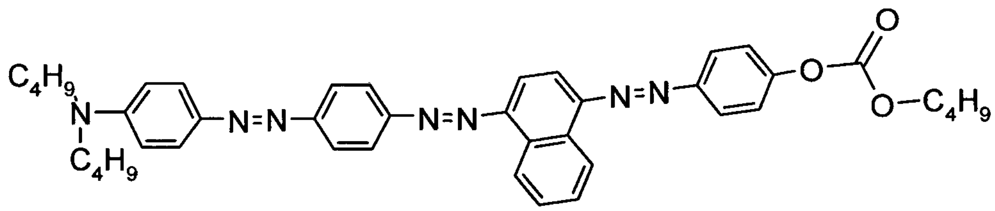 |
| D-3 |

Tabelle: Physikalische Eigenschaften der Farbstoffe

| Farbstoff | Hostmischung | Farbe | Bande No | Konz. in % | Schichtdicke in µm | λ_Max nm | ε_iso | R |
|---|---|---|---|---|---|---|---|---|
| D-1 | M-1 | rotviolett | 1 | 0.25 | 24.3 | 595 | 653 | 0.71 |
| | | | 2 | | | 425 | 148 | -0.07 |
| | | | | | | | | |
| Verb. (1) | M-1 | grün | 1 | 0.25 | 23.1 | 698 | 343 | 0.68 |
| | | | 2 | | | 417 | 503 | 0.72 |
| | | | | | | | | |
| Verb. (8) | M-1 | blau | 1 | 0.25 | 23.8 | 616 | 324 | 0.77 |
| | | | 2 | | | 370 | 768 | 0.74 |
| | | | | | | | | |
| Verb. (9) | M-1 | grün | 1 | 0.25 | 23.6 | 717 | 335 | 0.71 |
| | | | 2 | | | 413 | 545 | 0.72 |

Die Messungen zeigen, dass die erfindungsgemäßen Thienothiadiazol-Verbindungen hervorragende Eigenschaften in Bezug auf den Anisotropiegrad R aufweisen. Insbesondere ist der beinahe gleich große Anisotropiegrad bei der zweiten kürzerwelligen Absorptionsbande hervorzuheben. Die Absorptionen sind sowohl bei der langwelligen, als auch bei der kürzerwelligen Bande positiv dichroitisch.

Die Vergleichssubstanz D-1 ist dahingegen positiv dichroitisch in der längerwelligen Absorptionsbande und leicht negativ dichroitisch in der kürzerwelligen Absorptionsbande. Es findet somit ein Wechsel des Dichroismus über die Wellenlängen hinweg statt. In einer schwarzen Mischung aus mehreren ausschließlich positiv dichroitischen Farbstoffen unter Einsatz von Verbindung D-1 würde dann dieser negative Beitrag von D-1 im Bereich der zweiten Bande zu einer Verringerung des potentiell erzielbaren Hubs in der Transmission einer Anzeige führen. Dies würde einen Nachteil darstellen, der durch die Bereitstellung der erfindungsgemäßen Verbindungen mit ihrer Gleichausrichtung des Dichroismus beseitigt wird.

Die erfindungsgemäßen Verbindungen zeigen abhängig vom Substitutionsmuster unterschiedliche Absorptionsfarben, so dass eine Mischung enthaltend zwei oder mehr der erfindungsgemäßen Farbstoffe bereits einen großen Teil des sichtbaren Spektrums abdeckt. Wird als weiterer Farbstoff ein rot absorbierender Farbstoff hinzugefügt, kann eine schwarze Mischung erhalten werden.

Einige Vertreter der erfindungsgemäßen Farbstoffe zeigen eine grüne Farbe, die sonst nur über die Kombination aus einem gelben und blauen Farbstoff zu erhalten ist. Der Einsatz von grünen Farbstoffen kann zusammen mit einem roten Farbstoff bereits schwarz ergeben, so dass die Möglichkeit besteht schwarze Mischungen aus bereits zwei unterschiedlichen Farbstoffen herzustellen.

**C) Herstellung von flüssigkristallinen Medien enthaltend die Farbstoffe**

C-1) Herstellung der LC-Farbstoffmischungen LC-1 bis LC-4

Das Beispiel zeigt die prinzipielle Herstellbarkeit von Lösungen der erfindungsgemäßen Verbindungen in flüssigkristallinen Mischungen. Es werden die folgenden Farbstoffe in den angegebenen Anteilen zur Grundmischung M-1 (s. u.) hinzugegeben und eine Lösung hergestellt:

| Tabelle: LC-Farbstoffmischungen LC-1 bis LC-4 | | |
|---|---|---|
| | Farbstoff | Anteil |
| LC-1 | D-1 | 0.25 Gew.-% |
| LC-2 | Verbindung (1) | 0.25 Gew.-% |
| LC-3 | Verbindung (8) | 0.25 Gew.-% |
| LC-4 | Verbindung (9) | 0.25 Gew.-% |

| Tabelle: Zusammensetzung der Hostmischung M-1 | | |
|---|---|---|
| Klärpunkt | 114.5 °C | |
| Delta-n | 0.1342 | |
| $n_e$ | 1.6293 | |
| $n_o$ | 1.4951 | |
| Zusammensetzung | Verbindung | |
| | CPG-3-F | Gew.-% |
| | CPG-5-F | 5 |
| | CPU-3-F | 5 |
| | CPU-5-F | 15 |
| | CP-3-N | 15 |
| | CP-5-N | 16 |
| | CCGU-3-F | 16 |
| | CGPC-3-3 | 7 |
| | CGPC-5-3 | 4 |
| | CGPC-5-5 | 4 |
| | CCZPC-3-3 | 4 |
| | CCZPC-3-4 | 3 |
| | CCZPC-3-5 | 3 |

**D) Verwendung von flüssigkristallinen Medien enthaltend den erfindungsgemäßen Farbstoff (1) in Vorrichtungen zur Regulierung des Energie-Durchtritts**

Um die Vorrichtungen herzustellen, wird die Flüssigkristallmischung enthaltend den Farbstoff (1) in den Zwischenraum der folgenden Schichtenanordnung gefüllt:

- Substratschicht
- ITO-Schicht
- Polyimid-Orientierungsschicht
- mit Spacern offengehaltener Zwischenraum
- Polyimid-Orientierungsschicht
- ITO-Schicht
- Substratschicht

Die Flüssigkristallschicht ist in dieser Anordnung planar mit antiparallelem Pretiltwinkel orientiert. Diese Orientierung wird durch zwei antiparallel zueinander geriebene Polyimidschichten erreicht. Die Dicke der flüssigkristallinen Schicht wird durch Spacer definiert und beträgt üblicherweise 25 μm.

Es werden Werte für den Lichttransmissionsgrad $\tau_V$ jeweils für den dunklen und den hellen Schaltzustand der Vorrichtung ermittelt und im Folgenden aufgeführt. Der helle Schaltzustand wird durch Anlegen einer Spannung erreicht, während der dunkle Schaltzustand ohne Spannung vorliegt. Weiterhin wird der Farbort der Vorrichtung (in CIE-Koordinaten) im Dunkel- und im Hellzustand ermittelt.

Die Messung erfolgt mit der Vorrichtung enthaltend das flüssigkristalline Medium mit Farbstoffen im Messstrahl und einer baugleichen Vorrichtung, entsprechend ohne die Farbstoffe im Referenzstrahl. Hierdurch werden Reflexions- und Absorptionsverluste der Zelle eliminiert.

Der Wert $\tau_V$ und die CIE-Koordinaten (x,y) sind wie folgt definiert: $\tau_V$= Lichttransmissionsgrad, bestimmt nach DIN EN410 Der Farbort (für weiß, grau schwarz) der hier zugrundegelegten Normlichtart D65 liegt bei x=0.3127 und y=0.3290 (Manfred Richter, Einführung in die Farbmetrik, zweite Auflage 1991, ISBN 3-11-008209-8). Die angegebenen Farborte (x,y) beziehen sich alle auf die Normlichtart D65 und den 2° Normalbeobachter nach CIE 1931.

**Vorrichtungsbeispiel 1:**

Es wird die folgende flüssigkristalline Mischung verwendet:

| Bestandteil | Anteil |
|---|---|
| M-1 | 98.601% |
| erfindungsgemäße Verbindung (1) | 0.638% |
| D-1 | 0.385% |
| D-2 | 0.376% |

Erhaltene Messwerte für die Vorrichtung:

- Dunkelzustand: x=0.313; y= 0.329; $\tau_V$ =35%

- Hellzustand: x=0.313; y=0.333; $\tau_V$ =66%

Im Beispiel zeigt sich eine genügende Löslichkeit der Farbstoffe im flüssigkristallinen Medium. Weiterhin zeigt das Beispiel, dass sich die Vorrichtung durch Anlegen einer Spannung von einem Dunkelzustand mit deutlich geringerer Lichttransmission zu einem Hellzustand mit deutlich erhöhter Lichttransmission schalten lässt.

**Vorrichtungsbeispiel 2:**

| Bestandteil | Anteil |
|---|---|
| M-1 | 98.981% |
| Verbindung (1) | 0.600 % |
| D-3 | 0.419% |

Erhaltene Messwerte für die Vorrichtung:

- Dunkelzustand: x=0.316; y= 0.330; $\tau_v$ =35%
- Hellzustand: x=0.315; y=0.333; $\tau_v$ =68.2%

Im Beispiel zeigt sich eine genügende Löslichkeit der Farbstoffe im flüssigkristallinen Medium. Weiterhin zeigt das Beispiel, dass sich die Vorrichtung durch Anlegen einer Spannung von einem Dunkelzustand mit deutlich geringerer Lichttransmission zu einem Hellzustand mit deutlich erhöhter Lichttransmission schalten lässt. Das Beispiel zeigt eine schwarze Mischung bestehend aus nur zwei Farbstoffen, einem grünen und einem roten Farbstoff.

## Patentansprüche

1. Vorrichtung zur Regulierung des Energie-Durchtritts von einem Außenraum in einen Innenraum, wobei die Vorrichtung eine Schaltschicht enthält, wobei die Schaltschicht eine oder mehrere Verbindungen der Formel (I) enthält:

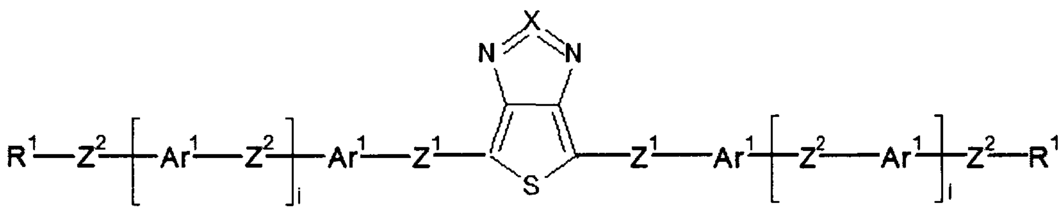

Formel (I),

wobei gilt:

X ist gleich S oder Se;
$Z^1$ ist unabhängig voneinander eine Einfachbindung, $-CR^3=CR^3-$ oder -C≡C-; oder zwei, drei, vier oder fünf miteinander kombinierte Gruppen, gewählt aus den Gruppen $-CR^3=CR^3-$ und -C≡C-;
$Z^2$ ist unabhängig voneinander eine Einfachbindung, O, S, $C(R^3)_2$, $-CR^3=CR^3-$ oder -C≡C-; oder zwei, drei, vier oder fünf miteinander kombinierte Gruppen, gewählt aus den Gruppen O, S, $C(R^3)_2$, $-CR^3=CR^3-$ und -C≡C-;
$Ar^1$ ist unabhängig voneinander eine Aryl- oder Heteroarylgruppe mit 5 bis 30 aromatischen Ringatomen, die mit einem oder mehreren Resten $R^4$ substituiert sein kann;
$R^1$ ist unabhängig voneinander H, D, F, CN, $N(R^5)_2$, oder eine Alkyl-, Alkoxy- oder Thioalkoxygruppe mit 1 bis 20 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C=CR^5-$, -C≡C-, C=O, C=S, -C(=O)O-, -OC(=O)-, $Si(R^5)_2$, $NR^5$, -O- oder -S- ersetzt sein können;
$R^3$, $R^4$ sind unabhängig voneinander H, D, F, Cl, CN, oder eine Alkyl-, Alkoxy-, oder Thioalkoxygruppe mit 1 bis 10 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C=CR^5-$, -C≡C-, C=O, C=S, -C(=O)O-, -OC(=O)-, $Si(R^5)_2$, $NR^5$, -O- oder -S- ersetzt sein können;
$R^5$ ist unabhängig voneinander H, D, F, Cl, CN, $N(R^6)_2$, eine Alkyl-, Alkoxy- oder Thioalkoxygruppe mit 1 bis 10 C-Atomen oder eine Alkenyl- oder Alkinylgruppe mit 2 bis 20 C-Atomen, wobei die oben genannten Gruppen jeweils mit einem oder mehreren Resten $R^6$ substituiert sein können und wobei eine oder mehrere $CH_2$-Gruppen in den oben genannten Gruppen durch $-R^6C=CR^6-$, -C≡C-, C=O, C=S, -C(=O)O-, -O(C=O)-, $Si(R^6)_2$, $NR^6$, -O- oder -S- ersetzt sein können, oder eine Aryl- oder Heteroarylgruppe mit 5 bis 30 aromatischen Ringatomen,

die jeweils mit einem oder mehreren Resten $R^6$ substituiert sein kann;
$R^6$ ist unabhängig voneinander H, F oder ein aliphatischer organischer Rest mit 1 bis 20 C-Atomen, in dem ein oder mehrere H-Atome durch F ersetzt sein können, oder eine Aryl- oder Heteroarylgruppe mit 5 bis 20 C-Atomen, in der ein oder mehrere H-Atome durch F ersetzt werden können; und
i ist unabhängig voneinander gleich 0, 1, 2, 3, 4 oder 5,

und wobei neben der Verbindung der Formel (I) in der Schaltschicht ein flüssigkristallines Medium enthaltend eine oder mehrere verschiedene Verbindungen vorliegt.

**2.** Vorrichtung nach Anspruch 1, **dadurch gekennzeichnet, dass** X gleich S ist, und/oder dass $Z^1$ eine Einfachbindung ist.

**3.** Vorrichtung nach einem oder mehreren der vorhergehenden Ansprüche, **dadurch gekennzeichnet, dass** $Z^2$ unabhängig voneinander für eine Einfachbindung, $-C(R^3)_2C(R^3)_2-$, $-CR^3=CR^3-$, $-C\equiv C-$, $-OC(R^3)_2-$ oder $-C(R^3)_2O-$ steht.

**4.** Vorrichtung nach einem oder mehreren der vorhergehenden Ansprüche, **dadurch gekennzeichnet, dass** $Ar^1$ unabhängig voneinander eine Arylgruppe mit 6 bis 15 C-Atomen oder eine Heteroarylgruppe mit 5 bis 15 C-Atomen darstellt, die mit einem oder mehreren Resten $R^4$ substituiert sein kann.

**5.** Vorrichtung nach einem oder mehreren der vorhergehenden Ansprüche, **dadurch gekennzeichnet, dass** $Ar^1$ bei jedem Auftreten gewählt ist aus wahlweise mit Resten $R^4$ substituiertem Benzol, Fluoren, Naphthalin, Pyridin, Pyrimidin, Pyrazin, Triazin, Thiophen, Thiophen mit ankondensiertem 1,4-Dioxanring, Benzothiophen, Dibenzothiophen, Benzodithiophen, Cyclopentadithiophen, Thienothiophen, Indenothiophen, Dithienopyrrol, Silolodithiophen, Selenophen, Benzoselenophen, Dibenzoselenophen, Furan, Benzofuran, Dibenzofuran und Chinolin.

**6.** Vorrichtung nach einem oder mehreren der vorhergehenden Ansprüche, **dadurch gekennzeichnet, dass** mindestens ein $Ar^1$ gewählt ist aus einer schwefelhaltigen Heteroarylgruppe, die mit einem oder mehreren Resten $R^4$ substituiert sein kann.

**7.** Vorrichtung nach einem oder mehreren der vorhergehenden Ansprüche, **dadurch gekennzeichnet, dass** $R^1$ unabhängig voneinander ausgewählt ist aus H, F, oder einer geradkettigen Alkyl- oder Alkoxylgruppe mit 3 bis 20 C-Atomen, die mit einem oder mehreren Resten $R^5$ substituiert sein kann, oder einer verzweigten Alkyl- oder Alkoxygruppe mit 3 bis 20 C-Atomen, die mit einem oder mehreren Resten $R^5$ substituiert sein kann, oder einer cyclischen Alkylgruppe mit 6 C-Atomen, die mit einem oder mehreren Resten $R^5$ substituiert sein kann, wobei in den Alkyl- und Alkoxygruppen eine oder mehrere $CH_2$-Gruppen durch -O-, -S- oder $-R^5C=CR^5-$ ersetzt sein können, oder einer Siloxanylgruppe mit 1 bis 6 Si-Atomen, die mit einem oder mehreren Resten $R^5$ substituiert sein kann.

**8.** Vorrichtung nach einem oder mehreren der vorhergehenden Ansprüche, **dadurch gekennzeichnet, dass** der Index i gleich 1 oder 2 ist.

**9.** Vorrichtung nach einem oder mehreren der vorhergehenden Ansprüche, **dadurch gekennzeichnet, dass** der Anisotropiegrad R der Verbindung der Formel (I), ermittelt wie in den Ausführungsbeispielen angegeben, größer ist als 0,4.

**10.** Vorrichtung nach einem oder mehreren der vorhergehenden Ansprüche, **dadurch gekennzeichnet, dass** sie elektrisch schaltbar ist.

**11.** Vorrichtung nach einem oder mehreren der vorhergehenden Ansprüche, **dadurch gekennzeichnet, dass** sie mit einer Solarzelle oder einer sonstigen Vorrichtung zur Umwandlung von Licht und/oder Wärmeenergie in elektrische Energie verbunden ist.

**12.** Fenster, enthaltend eine Vorrichtung nach einem oder mehreren der Ansprüche 1 bis 11.

**13.** Verbindung der Formel (I):

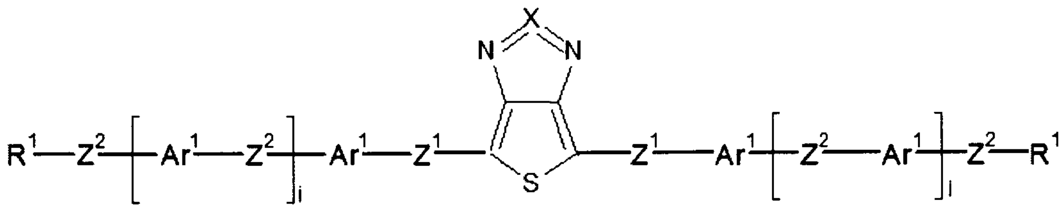

Formel (I),

wobei gilt:

X ist gleich S oder Se;
$Z^1$ ist unabhängig voneinander eine Einfachbindung, $-CR^3=CR^3-$ oder -C≡C-; oder zwei, drei, vier oder fünf miteinander kombinierte Gruppen, gewählt aus den Gruppen $-CR^3=CR^3-$ und -C≡C-;
$Z^2$ ist unabhängig voneinander eine Einfachbindung, O, S, $C(R^3)_2$, $-CR^3=CR^3-$ oder -C≡C-; oder zwei, drei, vier oder fünf miteinander kombinierte Gruppen, gewählt aus den Gruppen O, S, $C(R^3)_2$, $-CR^3=CR^3-$ und -C≡C-;
$Ar^1$ ist unabhängig voneinander eine Aryl- oder Heteroarylgruppe mit 5 bis 30 aromatischen Ringatomen, die mit einem oder mehreren Resten $R^4$ substituiert sein kann;
$R^1$ ist unabhängig voneinander eine geradkettige Alkyl- oder Alkoxygruppe mit 3 bis 10 C-Atomen oder eine verzweigte Alkyl- oder Alkoxygruppe mit 3 bis 10 C-Atomen;
$R^3$, $R^4$ sind unabhängig voneinander H, D, F, Cl, CN, oder eine Alkyl-, Alkoxy-, oder Thioalkoxygruppe mit 1 bis 10 C-Atomen, die mit einem oder mehreren Resten $R^5$ substituiert sein kann, wobei eine oder mehrere $CH_2$-Gruppen in den Alkyl-, Alkoxy- oder Thioalkoxygruppen durch $-R^5C=CR^5-$, -C≡C-, C=O, C=S, -C(=O)O-, -OC(=O)-, $Si(R^5)_2$, $NR^5$, -O- oder -S- ersetzt sein können;
$R^5$ ist unabhängig voneinander H, D, F, Cl, CN, $N(R^6)_2$, eine Alkyl-, Alkoxy- oder Thioalkoxygruppe mit 1 bis 10 C-Atomen oder eine Alkenyl- oder Alkinylgruppe mit 2 bis 20 C-Atomen, wobei die oben genannten Gruppen jeweils mit einem oder mehreren Resten $R^6$ substituiert sein können und wobei eine oder mehrere $CH_2$-Gruppen in den oben genannten Gruppen durch $-R^6C=CR^6-$, -C≡C-, C=O, C=S, -C(=O)O-, -O(C=O)-, $Si(R^6)_2$, $NR^6$, -O- oder -S- ersetzt sein können, oder eine Aryl- oder Heteroarylgruppe mit 5 bis 30 aromatischen Ringatomen, die jeweils mit einem oder mehreren Resten $R^6$ substituiert sein kann;
$R^6$ ist unabhängig voneinander H, F oder ein aliphatischer organischer Rest mit 1 bis 20 C-Atomen, in dem ein oder mehrere H-Atome durch F ersetzt sein können, oder eine Aryl- oder Heteroarylgruppe mit 5 bis 20 C-Atomen, in der ein oder mehrere H-Atome durch F ersetzt werden können; und
i ist unabhängig voneinander gleich 0, 1, 2, 3, 4 oder 5.

**14.** Flüssigkristallines Medium enthaltend eine Verbindung nach Anspruch 13.

**15.** Verwendung eines flüssigkristallinen Mediums gemäß Anspruch 14 in einem Fenster.

## Claims

**1.** Device for regulating the passage of energy from an outside space into an inside space, where the device contains a switching layer, where the switching layer comprises one or more compounds of the formula (I):

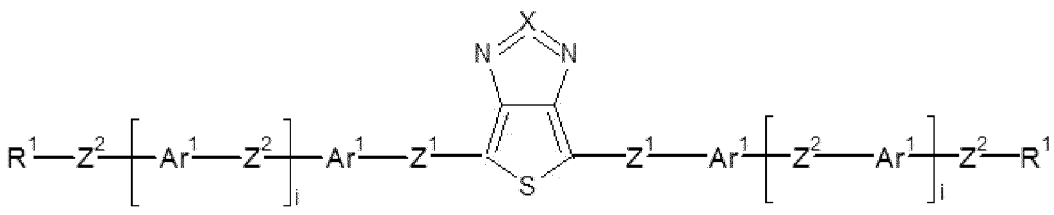

formula (I),

where:

X is equal to S or Se;

$Z^1$ is, independently of one another, a single bond, $-CR^3=CR^3-$ or $-C\equiv C-$; or two, three, four or five groups selected from the groups $-CR^3=CR^3-$ and $-C\equiv C-$ combined with one another;

$Z^2$ is, independently of one another, a single bond, O, S, $C(R^3)_2$, $-CR^3=CR^3-$ or $-C\equiv C-$; or two, three, four or five groups selected from the groups O, S, $C(R^3)_2$, $-CR^3=CR^3-$ and $-C\equiv C-$ combined with one another;

$Ar^1$ is, independently of one another, an aryl or heteroaryl group having 5 to 30 aromatic ring atoms, which may be substituted by one or more radicals $R^4$;

$R^1$ is, independently of one another, H, D, F, CN, $N(R^5)_2$, or an alkyl, alkoxy or thioalkoxy group having 1 to 20 C atoms, which may be substituted by one or more radicals $R^5$, where one or more $CH_2$ groups in the alkyl, alkoxy or thioalkoxy groups may be replaced by $-R^5C=CR^5-$, -C=C-, C=O, C=S, -C(=O)O-, -OC(=O)-, $Si(R^5)_2$, $NR^5$, -O- or -S-;

$R^3$, $R^4$ are, independently of one another, H, D, F, Cl, CN, or an alkyl, alkoxy or thioalkoxy group having 1 to 10 C atoms, which may be substituted by one or more radicals $R^5$, where one or more $CH_2$ groups in the alkyl, alkoxy or thioalkoxy groups may be replaced by $-R^5C=CR^5-$, $-C\equiv C-$, C=O, C=S, -C(=O)O-, -OC(=O)-, $Si(R^5)_2$, $NR^5$, -O- or -S-;

$R^5$ is, independently of one another, H, D, F, Cl, CN, $N(R^6)_2$, an alkyl, alkoxy or thioalkoxy group having 1 to 10 C atoms or an alkenyl or alkynyl group having 2 to 20 C atoms, where the above-mentioned groups may in each case be substituted by one or more radicals $R^6$ and where one or more $CH_2$ groups in the above-mentioned groups may be replaced by $-R^6C=CR^6-$, $-C\equiv C-$, C=O, C=S, -C(=O)O-, -O(C=O)-, $Si(R^6)_2$, $NR^6$, -O- or -S-, or an aryl or heteroaryl group having 5 to 30 aromatic ring atoms, which may in each case be substituted by one or more radicals $R^6$;

$R^6$ is, independently of one another, H, F or an aliphatic organic radical having 1 to 20 C atoms, in which one or more H atoms may be replaced by F, or an aryl or heteroaryl group having 5 to 20 C atoms, in which one or more H atoms may be replaced by F; and

i is, independently of one another, equal to 0, 1, 2, 3, 4 or 5,

and where a liquid-crystalline medium comprising one or more different compounds is present in the switching layer besides the compound of the formula (I).

2. Device according to Claim 1, **characterised in that** X is equal to S, and/or **in that** $Z^1$ is a single bond.

3. Device according to one or more of the preceding claims, **characterised in that** $Z^2$ stands, independently of one another, for a single bond, $-C(R^3)_2C(R^3)_2-$, $-CR^3=CR^3-$, $-C\equiv C-$, $-OC(R^3)_2-$ or $-C(R^3)_2O-$.

4. Device according to one or more of the preceding claims, **characterised in that** $Ar^1$ represents, independently of one another, an aryl group having 6 to 15 C atoms or a heteroaryl group having 5 to 15 C atoms, which may be substituted by one or more radicals $R^4$.

5. Device according to one or more of the preceding claims, **characterised in that** $Ar^1$ is selected on each occurrence from benzene, fluorene, naphthalene, pyridine, pyrimidine, pyrazine, triazine, thiophene, thiophene with condensed-on 1,4-dioxane ring, benzothiophene, dibenzothiophene, benzodithiophene, cyclopentadithiophene, thienothiophene, indenothiophene, dithienopyrrole, silolodithiophene, selenophene, benzoselenophene, dibenzoselenophene, furan, benzofuran, dibenzofuran and quinoline, each of which is optionally substituted by radicals $R^4$.

6. Device according to one or more of the preceding claims, **characterised in that** at least one $Ar^1$ is selected from a sulfur-containing heteroaryl group, which may be substituted by one or more radicals $R^4$.

7. Device according to one or more of the preceding claims, **characterised in that** $R^1$ is selected, independently of one another, from H, F, or a straight-chain alkyl or alkoxy group having 3 to 20 C atoms, which may be substituted by one or more radicals $R^5$, or a branched alkyl or alkoxy group having 3 to 20 C atoms, which may be substituted by one or more radicals $R^5$, or a cyclic alkyl group having 6 C atoms, which may be substituted by one or more radicals $R^5$, where one or more $CH_2$ groups in the alkyl and alkoxy groups may be replaced by -O-, -S- or $-R^5C=CR^5-$, or a siloxanyl group having 1 to 6 Si atoms, which may be substituted by one or more radicals $R^5$.

8. Device according to one or more of the preceding claims, **characterised in that** the index i is equal to 1 or 2.

9. Device according to one or more of the preceding claims, **characterised in that** the degree of anisotropy R of the compound of the formula (I), determined as indicated in the working examples, is greater than 0.4.

10. Device according to one or more of the preceding claims, **characterised in that** it is electrically switchable.

11. Device according to one or more of the preceding claims, **characterised in that** it is connected to a solar cell or another device for conversion of light and/or heat energy into electrical energy.

12. Window containing a device according to one or more of Claims 1 to 11.

13. Compound of the formula (I):

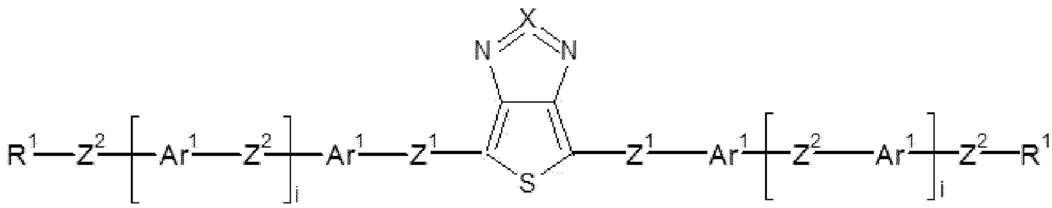

formula (I),

where:

X is equal to S or Se;
$Z^1$ is, independently of one another, a single bond, $-CR^3=CR^3-$ or $-C\equiv C-$; or two, three, four or five groups selected from the groups $-CR^3=CR^3-$ and $-C\equiv C-$ combined with one another;
$Z^2$ is, independently of one another, a single bond, O, S, $C(R^3)_2$, $-CR^3=CR^3-$ or $-C\equiv C-$; or two, three, four or five groups selected from the groups O, S, $C(R^3)_2$, $-CR^3=CR^3-$ and $-C\equiv C-$ combined with one another;
$Ar^1$ is, independently of one another, an aryl or heteroaryl group having 5 to 30 aromatic ring atoms, which may be substituted by one or more radicals $R^4$;
$R^1$ is, independently of one another, a straight-chain alkyl or alkoxy group having 3 to 10 C atoms or a branched alkyl or alkoxy group having 3 to 10 C atoms;
$R^3$, $R^4$ are, independently of one another, H, D, F, Cl, CN, or an alkyl, alkoxy or thioalkoxy group having 1 to 10 C atoms, which may be substituted by one or more radicals $R^5$, where one or more $CH_2$ groups in the alkyl, alkoxy or thioalkoxy groups may be replaced by $-R^5C=CR^5-$, $-C\equiv C-$, C=O, C=S, -C(=O)O-, -OC(=O)-, $Si(R^5)_2$, $NR^5$, -O- or -S-;
$R^5$ is, independently of one another, H, D, F, Cl, CN, $N(R^6)_2$, an alkyl, alkoxy or thioalkoxy group having 1 to 10 C atoms or an alkenyl or alkynyl group having 2 to 20 C atoms, where the above-mentioned groups may in each case be substituted by one or more radicals $R^6$ and where one or more $CH_2$ groups in the above-mentioned groups may be replaced by $-R^6C=CR^6-$, $-C\equiv C-$, C=O, C=S, -C(=O)O-, -O(C=O)-, $Si(R^6)_2$, $NR^6$, -O- or -S-, or an aryl or heteroaryl group having 5 to 30 aromatic ring atoms, which may in each case be substituted by one or more radicals $R^6$;
$R^6$ is, independently of one another, H, F or an aliphatic organic radical having 1 to 20 C atoms, in which one or more H atoms may be replaced by F, or an aryl or heteroaryl group having 5 to 20 C atoms, in which one or

more H atoms may be replaced by F; and
i is, independently of one another, equal to 0, 1, 2, 3, 4 or 5.

**14.** Liquid-crystalline medium comprising a compound according to Claim 13.

**15.** Use of a liquid-crystalline medium according to Claim 14 in a window.

## Revendications

**1.** Dispositif pour réguler le passage de l'énergie depuis un espace extérieur à l'intérieur d'un espace intérieur, dans lequel le dispositif contient une couche de commutation, dans lequel la couche de commutation comprend un ou plusieurs composé(s) de la formule (I) :

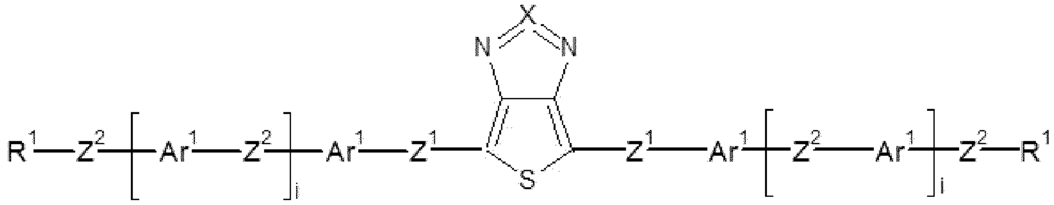

formule (I),

dans laquelle :

X est égal à S ou Se ;
$Z^1$ est, de manière indépendante pour chaque occurrence, une liaison simple, $-CR^3=CR^3-$ ou $-C\equiv C-$ ; ou deux, trois, quatre ou cinq groupes sélectionnés parmi les groupes $-CR^3=CR^3-$ et $-C\equiv C-$ en combinaison l'un avec l'autre ou les uns avec les autres ;
$Z^2$ est, de manière indépendante pour chaque occurrence, une liaison simple, O, S, $C(R^3)_2$, $-CR^3=CR^3-$ ou $-C\equiv C-$ ; ou deux, trois, quatre ou cinq groupes sélectionnés parmi les groupes O, S, $C(R^3)_2$, $-CR^3=CR^3-$ et $-C\equiv C-$ en combinaison l'un avec l'autre ou les uns avec les autres ;
$Ar^1$ est, de manière indépendante pour chaque occurrence, un groupe aryle ou hétéroaryle qui comporte de 5 à 30 atomes de cycle aromatique, lequel peut être substitué par un radical ou par plusieurs radicaux $R^4$ ;
$R^1$ est, de manière indépendante pour chaque occurrence, H, D, F, CN, $N(R^5)_2$, ou un groupe alkyle, alcoxy ou thioalcoxy qui comporte de 1 à 20 atome(s) de C, lequel peut être substitué par un radical ou par plusieurs radicaux $R^5$, où un ou plusieurs groupe(s) $CH_2$ dans les groupes alkyle, alcoxy ou thioalcoxy peut/peuvent être remplacé(s) par $-R^5C=CR^5-$, $-C\equiv C-$, C=O, C=S, -C(=O)O-, -OC(=O)-, $Si(R^5)_2$, $NR^5$, -O- ou -S -;
$R^3$, $R^4$ sont, de manière indépendante l'un de l'autre, H, D, F, Cl, CN, ou un groupe alkyle, alcoxy ou thioalcoxy qui comporte de 1 à 10 atome(s) de C, lequel peut être substitué par un radical ou par plusieurs radicaux $R^5$, où un ou plusieurs groupe(s) $CH_2$ dans les groupes alkyle, alcoxy ou thioalcoxy peut/peuvent être remplacé(s) par $-R^5C=CR^5-$, $-C\equiv C-$, C=O, C=S, -C(=O)O-, -OC(=O)-, $Si(R^5)_2$, $NR^5$, -O- ou -S- ;
$R^5$ est, de manière indépendante pour chaque occurrence, H, D, F, Cl, CN, $N(R^6)_2$, un groupe alkyle, alcoxy ou thioalcoxy qui comporte de 1 à 10 atome(s) de C ou un groupe alkényle ou alkynyle qui comporte de 2 à 20 atomes de C, où les groupes qui ont été mentionnés ci-avant peuvent dans chaque cas être substitués par un radical ou par plusieurs radicaux $R^6$ et où un ou plusieurs groupe(s) $CH_2$ dans les groupes qui ont été mentionnés ci-avant peut/peuvent être remplacé(s) par $-R^6C=CR^6-$, $-C\equiv C-$, C=O, C=S, -C(=O)O-, -O(C=O)-, $Si(R^6)_2$, $NR^6$, -O- ou -S-, ou un groupe aryle ou hétéroaryle qui comporte de 5 à 30 atomes de cycle aromatique, lequel peut dans chaque cas être substitué par un radical ou par plusieurs radicaux $R^6$ ;
$R^6$ est, de manière indépendante pour chaque occurrence, H, F ou un radical organique aliphatique qui comporte de 1 à 20 atome(s) de C, où un ou plusieurs atome(s) de H peut/ peuvent être remplacé(s) par F, ou un groupe aryle ou hétéroaryle qui comporte de 5 à 20 atomes de C, où un ou plusieurs atome(s) de H peut/peuvent être remplacé(s) par F ; et
i est, de manière indépendante pour chaque occurrence, égal à 0, 1, 2, 3, 4 ou 5 ;

et où un milieu cristallin liquide qui comprend un ou plusieurs composé(s) différent(s) est présent dans la couche de commutation en plus du composé de la formule (I).

2. Dispositif selon la revendication 1, **caractérisé en ce que** X est égal à S, et/ou **en ce que** $Z^1$ est une liaison simple.

3. Dispositif selon une ou plusieurs des revendications précédentes, **caractérisé en ce que** $Z^2$ représente, de manière indépendante pour chaque occurrence, une liaison simple, $-C(R^3)_2C(R^3)_2-$, $-CR^3=CR^3-$, $-C\equiv C-$, $-OC(R^3)_2-$ ou $-C(R^3)_2O-$.

4. Dispositif selon une ou plusieurs des revendications précédentes, **caractérisé en ce que** $Ar^1$ représente, de manière indépendante pour chaque occurrence, un groupe aryle qui comporte de 6 à 15 atomes de C ou un groupe hétéroaryle qui comporte de 5 à 15 atomes de C, lequel peut être substitué par un radical ou par plusieurs radicaux $R^4$.

5. Dispositif selon une ou plusieurs des revendications précédentes, **caractérisé en ce que** $Ar^1$ est sélectionné pour chaque occurrence parmi le benzène, le fluorène, le naphtalène, la pyridine, la pyrimidine, la pyrazine, la triazine, le thiophène, le thiophène avec un cycle 1,4-dioxane condensé dessus, le benzothiophène, le dibenzothiophène, le benzodithiophène, le cyclopentadithiophène, le thiénothiophène, l'indénothiophène, le dithiénopyrrole, le silolodithiophène, le sélénophène, le benzosélénophène, le dibenzosélénophène, le furane, le benzofurane, le dibenzofurane et la quinoline, dont chacun est en option substitué par des radicaux $R^4$.

6. Dispositif selon une ou plusieurs des revendications précédentes, **caractérisé en ce qu'**au moins un $Ar^1$ est sélectionné parmi un groupe hétéroaryle qui contient du soufre, lequel peut être substitué par un radical ou par plusieurs radicaux $R^4$.

7. Dispositif selon une ou plusieurs des revendications précédentes, **caractérisé en ce que** $R^1$ est sélectionné, de manière indépendante pour chaque occurrence, parmi H, F, ou un groupe alkyle ou alcoxy en chaîne droite qui comporte de 3 à 20 atomes de C, lequel peut être substitué par un radical ou par plusieurs radicaux $R^5$, ou un groupe alkyle ou alcoxy ramifié qui comporte de 3 à 20 atomes de C, lequel peut être substitué par un radical ou par plusieurs radicaux $R^5$, ou un groupe alkyle cyclique qui comporte 6 atomes de C, lequel peut être substitué par un radical ou par plusieurs radicaux $R^5$, où un ou plusieurs groupe(s) $CH_2$ dans les groupes alkyle et alcoxy peut/peuvent être remplacé(s) par -O-, -S- ou $-R^5C=CR^5-$, ou un groupe siloxanyle qui comporte de 1 à 6 atome(s) de Si, lequel peut être substitué par un radical ou par plusieurs radicaux $R^5$.

8. Dispositif selon une ou plusieurs des revendications précédentes, **caractérisé en ce que** l'indice i est égal à 1 ou 2.

9. Dispositif selon une ou plusieurs des revendications précédentes, **caractérisé en ce que** le degré d'anisotropie R du composé de la formule (I), lequel est déterminé comme indiqué au niveau des exemples de travail, est supérieur à 0,4.

10. Dispositif selon une ou plusieurs des revendications précédentes, **caractérisé en ce qu'**il peut être commuté électriquement.

11. Dispositif selon une ou plusieurs des revendications précédentes, **caractérisé en ce qu'**il est connecté à une cellule solaire ou à un autre dispositif pour la conversion de l'énergie lumineuse et/ou de l'énergie thermique en énergie électrique.

12. Vitre ou fenêtre contenant un dispositif selon une ou plusieurs des revendications 1 à 11.

13. Composé de la formule (I) :

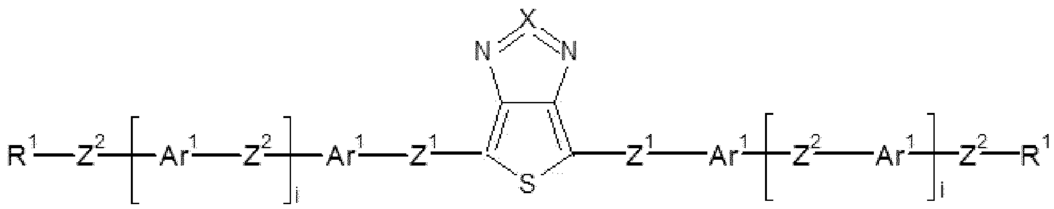

formule (I),

dans laquelle :

X est égal à S ou Se ;

$Z^1$ est, de manière indépendante pour chaque occurrence, une liaison simple, $-CR^3=CR^3-$ ou $-C\equiv C-$ ; ou deux, trois, quatre ou cinq groupes sélectionnés parmi les groupes $-CR^3=CR^3-$ et $-C\equiv C-$ en combinaison l'un avec l'autre ou les uns avec les autres ;

$Z^2$ est, de manière indépendante pour chaque occurrence, une liaison simple, O, S, $C(R^3)_2$, $-CR^3=CR^3-$ ou $-C\equiv C-$ ; ou deux, trois, quatre ou cinq groupes sélectionnés parmi les groupes O, S, $C(R^3)_2$, $-CR^3=CR^3-$ et $-C\equiv C-$ en combinaison l'un avec l'autre ou les uns avec les autres ;

$Ar^1$ est, de manière indépendante pour chaque occurrence, un groupe aryle ou hétéroaryle qui comporte de 5 à 30 atomes de cycle aromatique, lequel peut être substitué par un radical ou par plusieurs radicaux $R^4$ ;

$R^1$ est, de manière indépendante pour chaque occurrence, un groupe alkyle ou alcoxy en chaîne droite qui comporte de 3 à 10 atomes de C ou un groupe alkyle ou alcoxy ramifié qui comporte de 3 à 10 atomes de C ;

$R^3$, $R^4$ sont, de manière indépendante l'un de l'autre, H, D, F, Cl, CN, ou un groupe alkyle, alcoxy ou thioalcoxy qui comporte de 1 à 10 atome(s) de C, lequel peut être substitué par un radical ou par plusieurs radicaux $R^5$, où un ou plusieurs groupe(s) $CH_2$ dans les groupes alkyle, alcoxy ou thioalcoxy peut/peuvent être remplacé(s) par $-R^5C=CR^5-$, $-C\equiv C-$, C=O, C=S, -C(=O)O-, -OC(=O)-, $Si(R^5)_2$, $NR^5$, -O- ou -S- ;

$R^5$ est, de manière indépendante pour chaque occurrence, H, D, F, Cl, CN, $N(R^6)_2$, un groupe alkyle, alcoxy ou thioalcoxy qui comporte de 1 à 10 atome(s) de C ou un groupe alkényle ou alkynyle qui comporte de 2 à 20 atomes de C, où les groupes qui ont été mentionnés ci-avant peuvent dans chaque cas être substitués par un radical ou par plusieurs radicaux $R^6$ et où un ou plusieurs groupe(s) $CH_2$ dans les groupes qui ont été mentionnés ci-avant peut/peuvent être remplacé(s) par $-R^6C=CR^6-$, $-C\equiv C-$, C=O, C=S, -C(=O)O-, -O(C=O)-,$Si(R^6)_2$, $NR^6$, -O- ou -S-, ou un groupe aryle ou hétéroaryle qui comporte de 5 à 30 atomes de cycle aromatique, lequel peut dans chaque cas être substitué par un radical ou par plusieurs radicaux $R^6$ ;

$R^6$ est, de manière indépendante pour chaque occurrence, H, F ou un radical organique aliphatique qui comporte de 1 à 20 atome(s) de C, où un ou plusieurs atome(s) de H peut/ peuvent être remplacé(s) par F, ou un groupe aryle ou hétéroaryle qui comporte de 5 à 20 atomes de C, où un ou plusieurs atome(s) de H peut/peuvent être remplacé(s) par F ; et

i est, de manière indépendante pour chaque occurrence, égal à 0, 1, 2, 3, 4 ou 5.

**14.** Milieu cristallin liquide comprenant un composé selon la revendication 13.

**15.** Utilisation d'un milieu cristallin liquide selon la revendication 14 dans une vitre ou fenêtre.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009141295 A
- US 20100259698 A
- WO 2015090506 A
- WO 2014090373 A
- WO 2004090046 A
- WO 2010031479 A1
- WO 2013102038 A
- EP 34832 A
- EP 44893 A
- EP 48583 A
- EP 54217 A
- EP 56492 A
- EP 59036 A
- GB 2065158 A
- GB 2065695 A
- GB 2081736 A
- GB 2082196 A
- GB 2094822 A
- GB 2094825 A
- JP OS55123673 A
- DE 3017877
- DE 3040102
- DE 3115147
- DE 3115762
- DE 3150803
- DE 3201120
- DE 3126108
- DE 3202761
- EP 43904 A
- DE 3123519
- WO 822054 A
- GB 2079770 A
- JP OS5657850 A
- JP OS56104984 A
- US 4308161 A
- US 4308162 A
- US 4340973 A
- EP 60895 A
- EP 68427 A
- WO 821191 A
- EP 2166040 A
- US 20110042651 A
- EP 47027 A
- DE 3110960
- EP 698649 A
- EP 12008320 A
- WO 2010118422 A
- US 7038745 B
- US 6099758 A
- US 20110043732 A
- US 7023602 B
- WO 2012052100 A


### In der Beschreibung aufgeführte Nicht-Patentliteratur


- **ZHANG et al.** *J. Material. Chem.,* 2004, vol. 14, 1901-1904
- **TANAKA et al.** *Heterocycles,* 1994, vol. 37 (2), 693-695
- **J. QI et al.** *Journal of Materials Chemistry C,* 2014, vol. 2, 2431-2438
- **J. KMINEK et al.** *Journal of Polymer Science A: Polymer Chemistry,* 2010, vol. 48, 2743-2756
- **J. A. MIKROYANNIDIS et al.** *Journal of Materials Chemistry,* 2011, vol. 21, 4679-4688
- **STEINBERGER et al.** *J. Mater. Chem.,* 2012, vol. 22, 2701-2712
- **SHARMA et al.** *RSC Advances,* 2012, vol. 2 (30), 11457-11464
- **LI et al.** *Dyes Pigm.,* 2011, vol. 92, 674-680
- **MANFRED RICHTER.** Einführung in die Farbmetrik. Verlag Walter de Gruyter & Co, 1981
- **B. BAHADUR.** Liquid Crystals - Applications and Uses. World Scientific Publishing, 1992, vol. 3
- **T. UCHIDA ; C. SHISHIDO ; H. SEKI ; M. WADA.** *Mol. Cryst. Liq. Cryst.,* 1977, vol. 39, 39-52
- **H. SEKI ; C. SHISHIDO ; S. YASUI ; T. UCHIDA.** *Jpn. J. Appl. Phys.,* 1982, vol. 21, 191-192
- **JOSEPH R. LAKOWICZ.** Principles of Fluorescence Spectroscopy. Verlag Springer Science+Business Media LLC, 2010, 279
- *Technical Digest SID,* 2006, vol. 45.1
- Merck Liquid Crystals, Physical Properties of Liquid Crystals. Merck KGaA, November 1997
- **D. S. KLIGER et al.** Polarized Light in Optics and Spectroscopy. Academic Press, 1990
- **MANFRED RICHTER.** Einführung in die Farbmetrik. 1991